# EUROPEAN PATENT SPECIFICATION

(11) **EP 1 682 532 B1**
(45) Date of publication and mention of the grant of the patent: **27.06.2007**
(21) Application number: 04769673.7
(22) Date of filing: 19.10.2004
(51) Int. Cl.: C07D 403/06, A61K 31/506, A61P 19/02

(54) **PYRIMIDINES AS INHIBITORS OF PHOSPHOINOSITIDE-3-KINASES (PI3K)**
PYRIMIDINE ALS INHIBITOREN VON PHOSPHOINOSITID-3-KINASEN (PI3K)
PYRIMIDINES A TITRE D'INHIBITEURS DE PHOSPHOINOSITIDE-3-KINASES (PI3K)

(30) Priority: 31.10.2003 US 516591 P
(43) Date of publication of application: 26.07.2006
(73) Proprietor: Warner-Lambert Company LLC, New York, NY 10017 (US)
(72) Inventor: CONNOLLY, Michael Kevin, Pfizer Global R & D, Ann Arbor, MI 48105 (US); GOGLIOTTI, Rocco Dean, Pfizer Global R & D, Ann Arbor, MI 48105 (US); PLUMMER, Mark Stephen, Pfizer Global R & D, Ann Arbor, MI 48105 (US); VISNICK, Melean, Pfizer Global R & D, Ann Arbor, MI 48105 (US)
(74) Representative: Dekker, Henrike Cornelie Christine
(86) International application number: PCT/IB2004/003417
(87) International publication number: WO 2005/042519

(56) References cited:
- WO-A-01/53266
- STEPHEN WARD ET AL: "Therapeutic Potential of Phosphoinositide 3-Kinase Inhibitors" CHEMISTRY AND BIOLOGY, CURRENT BIOLOGY, LONDON, GB, vol. 10, no. 3, March 2003 (2003-03), pages 207-213, XP002289830 ISSN: 1074-5521

## Description

Phosphoinositide-3-kinases (PI3Ks) are a family of lipid kinases that phosphorylate phosphoinositols on the 3'-OH to generate PI-3-P (phosphatidylinositol 3-phosphate), PI-3,4-P2 and PI-3,4,5-P3. One class of PI3Ks are stimulated by growth factors. A separate class of PI3Ks are activated by G-protein coupled receptors and include PI3Kγ. The growth-factor stimulated PI3Ks (e.g., PI3Kα), have been implicated in cellular proliferation and cancer. PI3Kγ has been demonstrated to be involved in signaling cascades. For example, PI3Kγ is activated in response to ligands such as C5a, fMLP, ADP, and IL-8. In addition, PI3Kγ has been implicated in immune diseases (Hirsch et al. Science 2000;287:1049-1053). PI3Kγ null macrophages show a reduced chemotactic response and a reduced ability to fight inflammation (Hirsch et al., 2000, supra). Furthermore, PI3Kγ has also been implicated in thrombolytic diseases (e.g., thromboembolism, ischemic diseases, heart attacks, and stroke) (Hirsch et al. FASEB J. 2000;15(11):2019-2021; and Hirsch et al. FASEB J., July 9 2001;10.1096/fj.00-0810fje (cited herein as Hirsch et al., 2001).

Inhibitors of members of the PI3Ks are being developed for the treatment of human disease (see e.g., WO 01/81346; WO 01/53266; and WO 01/83456). There is a need for additional compounds that can inhibit PI3Ks for use as pharmaceutical agents:

### SUMMARY OF THE INVENTION

In one aspect, the present invention provides for pyrimidines of formula I: 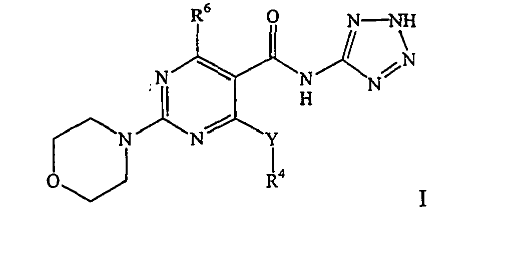 or a pharmaceutically acceptable salt thereof;
wherein:
R⁶ is hydrogen or methyl;
Y is O, NH, or S;
R⁴ is selected from the group consisting of: a C₂-C₆ alkyl, a -L-C₃-C₈ cycloalkyl, an adamantyl, a 5 or 6-membered heterocycloalkyl, and -J-R^{c};
   wherein R^{c} is a phenyl group or a naphthyl group,
   wherein L is absent or a C₁-C₃alkylene, and
   wherein J is absent or is a C₁-C₄-alkylene.

In certain embodiments of Formula I, Y is O, R⁶ is hydrogen, and R⁴ is a C₃-C₈ cycloalkyl-a compound of Formula II:

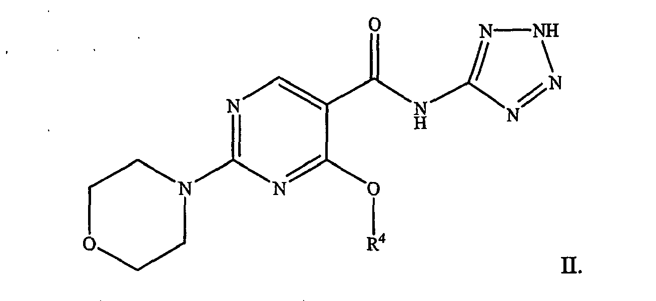

Examples of a compound of Formula II include, but are not limited to:
4-Cycloheptyloxy-2-morpholin-4-yl-pyrimidine-5-carboxylic acid (2H-tetrazol-5-yl)-amide;
4-Cyclopentyloxy-2-morpholin-4-yl-pyrimidine-5-carboxylic acid (2H-tetrazol-5-yl)-amide;
4-Cyclohexyloxy-2-morpholin-4-yl-pyrimidine-5-carboxylic acid (2H-tetrazol-5-yl)-amide;
4-Cyclooctyloxy-2-morpholin-4-yl-pyrimidine-5-carboxylic acid (2H-tetrazol-5-yl)-amide;
4-(2-Cyclohexyl-ethoxy)-2-morpholin-4-yl-pyrimidine-5-carboxylic acid (2H-tetrazol-5-yl)-amide;
4-Cyclohexylmethoxy-2-morpholin-4-yl-pyrimidine-5-carboxylic acid (2H-tetrazol-5-yl)-amide;
4-(3,3-Dimethyl-cyclohexyloxy)-2-morpholin-4-yl-pyrimidine-5-carboxylic acid (2H-tetrazol-5-yl)-amide; and
4-(3,5-Dimethyl-cyclohexyloxy)-2-morpholin-4-yl-pyrimidine-5-carboxylic acid (2H-tetrazol-5-yl)-amide.

In certain embodiments of Formula I, Y is O, R⁶ is methyl, and R⁴ is a C₃-C₈ cycloalkyl-a compound of Formula III:

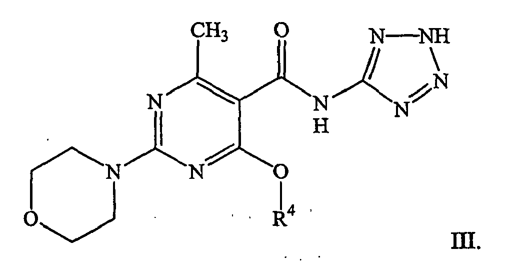

An example of a compound of Formula III is:
4-cycloheptyloxy-6-methyl-2-morpholin-4-yl-pyrimidine-5-carboxylic acid (2H-tetrazol-5-yl)-amide.

In certain embodiments of Formula I, Y is NH, and R⁶ is hydrogen-a compound of Formula IV:

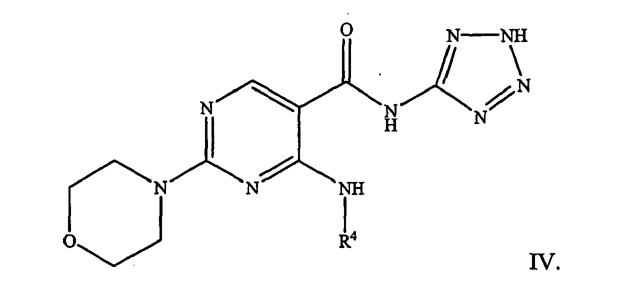

Examples of a compound of Formula IV include, but are not limited to:
4-Cyclopentylamino-2-morpholin-4-yl-pyrimidine-5-carboxylic acid (1H-tetrazol-5-yl)-amide;
4-Benzylamino-2-morpholin-4-yl-pyrimidine-5-carboxylic acid (1H-tetrazol-5-yl)-amide; and
4-Cyclohexylamino-2-morpholin-4-yl-pyrimidine-5-carboxylic acid (1H-tetrazol-5-yl)-amide.

In certain embodiments of Formula I, Y is S -a compound of Formula V:

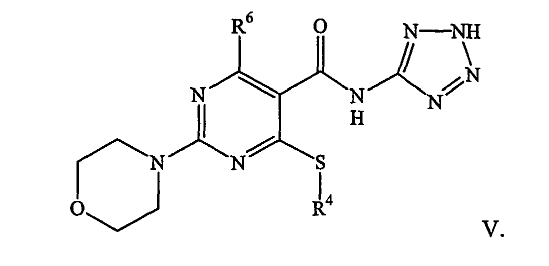

An example of a compound of Formula V is:
4-cycloheptylsulfanyl-2-morpholin-4-yl-pyrimidine-5-carboxylic acid (2H-tetrazol-5-yl)-amide.

In another aspect, the invention provides for pharmaceutical compositions that comprise a therapeutically effective amount of a compound of any one of Formulas I-V and a pharmaceutically acceptable carrier. In certain embodiments, these compositions are useful in the treatment of a PI3K-mediated disorder or condition. The compounds of the invention can also be combined in a pharmaceutical composition that also comprise compounds that are useful for the treatment of cancer, a thrombolytic disease, heart disease, stroke, an inflammatory disease such as rheumatoid arthritis, or another PI3K-mediated disorder.

In another aspect, the present invention provides for methods of treating a subject suffering from a PI3K-mediated disorder or condition comprising:
administering, to a subject suffering from a PI3K-mediated condition or disorder, a pharmaceutical composition comprising a therapeutically effective amount of a compound of any one of Formulas I-V and a pharmaceutically acceptable carrier. In certain embodiments, the PI3K-mediated condition or disorder is selected from the group consisting of: rheumatoid arthritis, ankylosing spondylitis, osteoarthritis, psoriatic arthritis, psoriasis, inflammatory diseases, and
autoimmune diseases. In other embodiments, the PI3K-mediated condition or disorder is selected from the group consisting of: cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, and coronary artery disease. In still other embodiments, the PI3K-mediated condition or disorder is selected from the group consisting of: cancer, colon cancer, glioblastoma, endometrial carcinoma, hepatocellular cancer, lung cancer, melanoma, renal cell carcinoma, thyroid carcinoma, cell lymphoma, lymphoproliferative disorders, small cell lung cancer, squamous cell lung carcinoma, glioma, breast cancer, prostate cancer, ovarian cancer, cervical cancer, and leukemia. In yet another embodiment, the PI3K-mediated condition or disorder is selected from the group consisting of: type II diabetes. In still other embodiments, the PI3K-mediated condition or disorder is selected from the group consisting of: respiratory diseases, bronchitis, asthma, and chronic obstructive pulmonary disease. In certain embodiments, the subject is a human.

### DEFINITIONS

As used herein, the following terms have the meanings ascribed to them unless specified otherwise.

A "PI3K-mediated disorder or condition" is characterized by the participation of one or more PI3Ks or a PI3P phosphatase, (e.g., PTEN, etc.) in the inception, manifestation of one or more symptoms or disease markers, severity, or progression of a disorder or condition. PI3K-mediated disorders and conditions include, but are not limited to: rheumatoid arthritis, ankylosing spondylitis, osteoarthritis, psoriatic arthritis, psoriasis, inflammatory diseases, pulmonary fibrosis, autoimmune diseases, cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, coronary artery disease, cancer, breast cancer, gliobastoma, endometrial carcinoma, hepatocellular carcinoma, colon cancer, lung cancer, melanoma, renal cell carcinoma, thyroid carcinoma, small cell lung cancer, squamous cell lung carcinoma, glioma, prostate cancer, ovarian cancer, cervical cancer, leukemia, cell lymphoma, lymphoproliferative disorders, type II diabetes, respiratory diseases, bronchitis, asthma, and chronic obstructive pulmonary disease.

A PI3K is an enzyme that is able to phosphorylate the 3'-OH of a phosphoinositol to generate PI3P. PI3Ks include, but are not limited to, PI3Kα, PI3Kβ, PI3Kγ, and PI3Kδ. A PI3K typically comprises at least one catalytic subunit (e.g., p110γ), and may further comprise a regulatory subunit (e.g., p101, etc.).

The term "alkyl group" or "alkyl" includes straight and branched carbon chain radicals. The term "alkylene" refers to a diradical of an unsubstituted or substituted alkane. For example, a "C₂₋₆ alkyl" is an alkyl group having from 2 to 6 carbon atoms. Examples of C₂-C₆ straight-chain alkyl groups include, but are not limited to, ethyl, n-propyl, n-butyl, n-pentyl, and n-hexyl. Examples of branched-chain alkyl groups include, but are not limited to, isopropyl, *tert*-butyl, isobutyl, etc. Examples of alkylene groups include, but are not limited to, -CH₂-, - CH₂-CH₂-, -CH₂-CH(CH₃)-CH₂-, and -(CH₂)₁₋₃. Alkylene groups can be substituted with groups as set forth below for alkyl.

The term alkyl includes both "unsubstituted alkyls" and "substituted alkyls," the latter of which refers to alkyl moieties having substituents replacing a hydrogen on one or more carbons of the hydrocarbon backbone. Such substituents are independently selected from the group consisting of: halo, I, Br, Cl, F, -OH, -COOH, trifluoromethyl, -NH₂, -OCF₃, and O-C₁-C₃ alkyl.

Typical substituted alkyl groups thus are 2,3-dichloropentyl, 3-hydroxy-5-carboxyhexyl, 2-aminopropyl; pentachlorobutyl, trifluoromethyl, methoxyethyl, 3-hydroxypentyl, 4-chlorobutyl, 1,2-dimethyl-propyl, and pentafluoroethyl.

"Halo" includes fluoro, chloro, bromo, and iodo.

The term "C₃-C₈cycloalkyl" refers to a cycloalkyl group containing from 3 to 8 carbons. Thus, the term "C₃-C₈cycloalkyl" encompasses monocyclic cycloalkyl groups containing from 3 to 8 carbons and bicyclic cycloalkyl groups containing 7 or 8 carbons. Examples of "C₃-C₈cycloalkyls" include, but are not limited to, cyclopropyl, cyclobutyl, cyclopentyl, cyclohexyl, cycloheptyl, and bicyclo[2.2.1]heptyl; the cycloalkyl group may optionally contain 1 or 2 double bonds (i.e., a cycloalkylenyl) including, but not limited to, cyclopentenyl, cyclohexenyl, and cycloheptenyl. A "C₃-C₈cycloalkyl" may be substituted with 1 or 2 groups independently selected from C₁-C₃alkyl (e.g., methyl) and -O-C₁-C₃alkyl (e.g., methoxy). Examples of substituted cycloalkyl groups include, but are not limited to, methyl-cyclopropyl, dimethyl-cyclohexyl, 2-methyl-cyclohexyl, 3-methyl-cyclohexyl, 3,5-dimethyl-cyclohexyl, and 4-methyl-cyclohexyl.

The term "adamantyl" includes unsubstituted adamantyl and adamantyls substituted with 1 or 2 groups independently selected from C₁-C₃alkyl (e.g., methyl) and -O-C₁-C₃alkyl (e.g., methoxy).

The phrase "5 or 6-membered heterocycloalkyl" means a stable cyclic group having carbon atoms and 1 to 3 heteroatoms independently selected from S, N or O, wherein when two O atoms or one O atom and one S atom are present, the two O atoms or one O atom and one S atom are not bonded directly to each other, respectively. Optionally, a 5 or 6-membered heterocycloalkyl may contain 1 or 2 carbon-carbon or carbon-nitrogen double bonds. Illustrative examples of 5 or 6-membered heterocycloalkyl include tetrahydrofuran-3-yl, morpholin-4-yl; tetrahydro-thiopyran-4-yl, piperidinyl, tetrahydropyranyl, and 4-methyl-piperazin-2-yl.

Unless otherwise indicated, the foregoing heterocycloalkyls can be C-attached or N-attached where such is possible and which results in the creation of a stable structure. For example, piperidinyl can be piperidin-1-yl (N-attached) or piperidin-4-yl (C-attached).

Embraced within the term "5 or 6 membered heterocycloalkyl" are 5 membered rings having one carbon-carbon or one carbon-nitrogen double bond in the ring (e.g., 2-pyrrolinyl, 3-pyrrolinyl, etc.) and 6 membered rings having one carbon-carbon or one carbon-nitrogen double bond in the ring (e.g., dihydro-2H-pyranyl, 1,2,3,4-tetrahydropyridine, 3,4-dihydro-2H-[1,4]oxazine, etc.).

A "5-membered heterocycloalkyl" is a stable 5-membered, monocyclic cycloalkyl ring having from 2 to 4 carbon atoms and from 1 to 3 heteroatoms selected from the group consisting of: 1 O; 1 S; 1 N; 2 N; 3 N; 1 S and 1 N; 1 S, and 2 N; 1 O and 1 N; and 1 O and 2 N. Illustrative examples of stable 5-membered heterocycloalkyls include tetrahydrofuranyl, dihydrofuranyl, tetrahydrothienyl, dihydrothienyl, imidazolidinyl, oxazolidinyl, imidazolinyl, isoxazolidinyl, pyrrolidinyl, 2-pyrrolinyl, and 3-pyrrolinyl.

A "6-membered heterocycloalkyl" is a stable 6-membered, monocyclic cycloalkyl ring having from 3 to 5 carbon atoms and from 1 to 3 heteroatoms selected from the group consisting of: 1 O; 2 O;1 S; 2 S; 1 N; 2 N; 3 N; 1 S, 1 O, and 1 N; 1 S and 1 N; 1 S and 2 N; 1 S and 1 O; 1 S and 2 O; 1 O and 1 N; and 1 O and 2 N. Illustrative examples of stable 6-membered heterocycloalkyls include tetrahydropyranyl, dihydropyranyl, dioxanyl, 1,3-dioxolanyl, 1,4-dithianyl, hexahydropyrimidine, morpholinyl, piperazinyl, piperidinyl, 2H-pyranyl, 4H-pyranyl, pyrazolidinyl, pyrazolinyl, 1,2,3,6-tetrahydropyridinyl, tetrahydrothiopyranyl, 1,1-dioxo-hexahydro-1λ⁶-thiopyranyl, 1, 1-dioxo-1λ⁶-thiomorpholinyl, thiomorpholinyl, thioxanyl, and trithianyl.

The term "5 or 6-membered heterocycloalkyl" includes saturated and unsaturated "5 or 6-membered heterocycloalkyls." "5 or 6-membered heterocycloalkyls" may be substituted such as those set out above for C₃-C₈cycloalkyls, where possible.

The term "phenyl" refers to unsubstituted and substituted phenyl groups. A phenyl group may be substituted with 1 to 3 substituents independently selected from the group consisting of: C₁-C₃alkyl, -O-C₁-C₃alkyl,-OCF₃, halo, and a C₅-C₆ cyloalkyl.

Typical substituted phenyl groups include, but are not limited to, 3-chlorophenyl, 2,6-dibromophenyl, 2,4,6-tribromophenyl, 2,6-dichlorophenyl, 4-trifluoromethylphenyl, 3-methyl-phenyl, 4-methyl-phenyl, 3,5-dimethyl-phenyl, 3,4,5-trimethoxy-phenyl, 3,5-dimethoxy-phenyl, 3,4-dimethoxy-phenyl, 3-methoxy-phenyl, 4-methoxy-phenyl, 3,5-difluoro-phenyl, 4-chloro-phenyl, 3-trifluoromethyl-phenyl, 3,5-dichloro-phenyl, 2-methoxy-5-methyl-phenyl, 2-fluoro-5-methyl-phenyl, 4-chloro-2-trifluoromethyl-phenyl, and the like.

A "naphthyl group" refers to unsubstituted and substituted naphthyl groups. A naphthyl group may be substituted with 1 to 4 substituents independently selected from the group consisting of: C₁-C₃alkyl, -O-C₁-C₃alkyl, - OCF₃, halo, and a C₅-C₆ cycloalkyl.

Some of the compounds in the present invention may exist as stereoisomers, including enantiomers, diastereomers, and geometric isomers. Geometric isomers include compounds of the present invention that have alkenyl groups, which may exist as entgegen or zusammen conformations, in which case all geometric forms thereof, both entgegen and zusammen, cis and *trans,* and mixtures thereof, are within the scope of the present invention. Some compounds of the present invention have cycloalkyl groups, which may be substituted at more than one carbon atom, in which case all geometric forms thereof, both *cis* and *trans,* and mixtures thereof, are within the scope of the present invention. All of these forms, including (R), (S), epimers, diastereomers, cis, trans, syn, anti, (E), (Z), tautomers, and mixtures thereof, are contemplated in the compounds of the present invention.

### DETAILED DESCRIPTION OF THE INVENTION

### I. INTRODUCTION

The present invention relates to pyrimidines of Formulas I-V, wherein R⁴, R⁶, and Y have any of the values defined therefor in the specification, and pharmaceutically acceptable salts thereof, that are useful as agents in the treatment of diseases and conditions, including inflammatory diseases, cardiovascular diseases, and cancers. Also provided are pharmaceutical compositions comprising one or more compounds of Formulas I-V.

### II. PREPARATION OF COMPOUNDS

Compounds of the present invention (e.g., compounds of Formulas I-V) can be prepared by applying synthetic methodology known in the art and synthetic methodology outlined in the schemes set forth below.

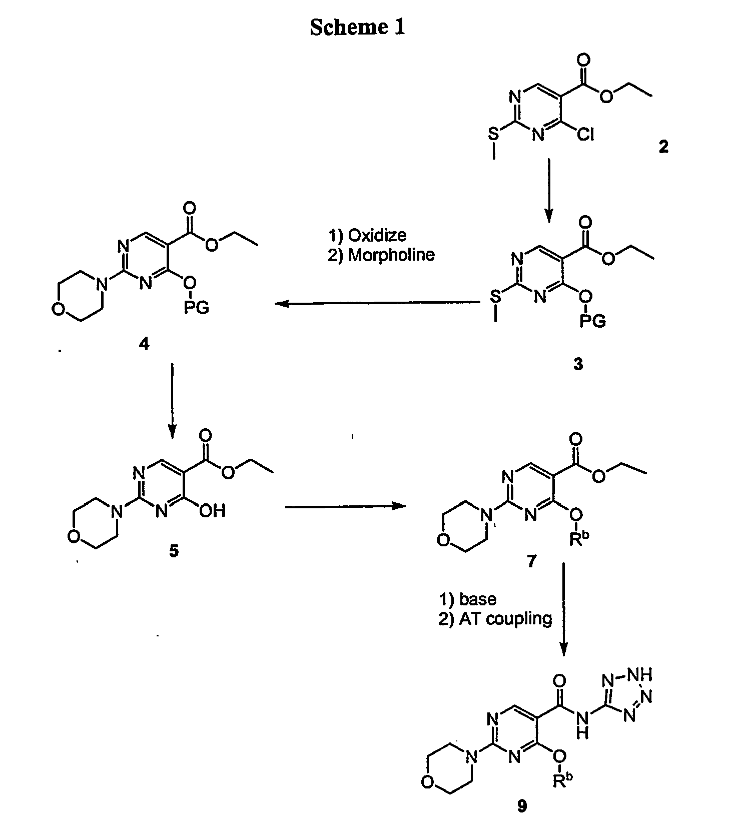

In Scheme **1, 2** (4-chloro-2-methylsulfanyl-pyrimidine-5-carboxylic acid ethyl ester; Sigma-Aldrich Co.) is reacted with a suitable hydroxyl protecting group reagent **PG** (e.g., (4-nitro-phenyl)-methanol) to provide **3** (e.g., 2-methylsulfanyl-4-(4-nitro-benzyloxy)-pyrimidine-5-carboxylic acid ethyl ester). Those of skill in the art will recognize that a wide variety of protecting groups can be used in Scheme 1 as a suitable protecting group for a hydroxyl group (see e.g., Greene and Wuts, Protective Groups in Organic Synthesis, 2nd ed., Chapter 2 (John Wiley & Sons, Inc., 1991)). For example, **2** can be reacted with an alkyl-lithium (e.g., sec-butyl-lithium, n-butyl-lithium) treated substituted or unsubstituted benzyl alcohol (e.g., (4-nitro-phenyl)-methanol) in a solvent such as THF (tetrahydrofuran) to provide **3** (e.g., 2-methylsulfanyl-4-(4-nitro-benzyloxy)-pyrimidine-5-carboxylic acid ethyl ester).

The methyl thio group of **3** is then oxidized to the corresponding methyl sulfinyl derivative using a suitable oxaziridine (e.g., Davis oxaziridine ((1S)-(+)-(10-camphorsulfonyl)oxaziridine); or 3-phenyl-2-(phenylsulfonyl)-1,2-oxaziridine, etc.), dimethyldioxirane, or mCPBA (3-chloroperoxybenzoic acid) in a solvent such as chloroform or dichloromethane. The methyl sulfinyl group is then displaced with morpholine to yield **4** (e.g., 2-morpholin-4-yl-4-(4-nitro-benzyloxy)-pyrimidine-5-carboxylic acid ethyl ester).

The hydroxyl protecting group on **4** is then removed to provide **5** (e.g., 4-Chloro-2-morpholin-4-yl-pyrimidine-5-carboxylic acid ethyl ester). For example a nitro-benzyloxy group can be cleaved to provide a hydroxyl via reduction with a palladium/carbon catalyst in an alcohol solvent such as ethanol under hydrogen gas at a suitable pressure. In certain embodiments if the protecting group is an optionally substituted phenyl or naphthyl group of R⁴, the protecting group is not removed. **4** is then treated with base and reacted with aminotetrazole as described below to provide **9.**

**5** is reacted with R^{b}-OH (e.g., cycloheptanol) in a Mitsunobu reaction with DEAD (diethyl azodicarboxylate) and PPh₃ (triphenylphoshine) in a solvent such as tetrahydrofuran (THF) to provide **7** (e.g., 4-cycloheptyloxy-2-morpholin-4-yl-pyrimidine-5-carboxylic acid ethyl ester). R^{b}-OH is a compound that includes but is not limited to a compound of formula HO-L-C₃-C₈ cycloalkyl (where L is absent or present), bicyclo[2.2.1]heptanol, adamantanol, wherein L is absent or a C₁-C₃alkylene. Examples of R^{b}-OH include, but are not limited to, cyclohexyl-methanol, cyclohexanol, phenyl-methanol, cycloheptanol, and cyclooctanol.

**7** is then saponified with an inorganic base (e.g., LiOH, NaOH, etc.) in MeOH and THF; dioxane; dioxane and water; or methanol and water, to provide the corresponding carboxylic acid **8** (e.g., 4-cycloheptyloxy-2-morpholin-4-yl-pyrimidine-5-carboxylic acid). The carboxylic acid is then coupled with 5-aminotetrazole using carbonyl diimidazole (CDI) in a non-protic solvent such as THF (tetrahydrofuran) to provide the carboxamide **9** (e.g., 4-cycloheptyloxy-2-morpholin-4-yl-pyrimidine-5-carboxylic acid (2H-tetrazol-5-yl)-amide). Alternatively, **8** in anhydrous CH₂Cl₂ can be treated with a catalytic amount of DMF followed by oxalyl chloride. 5-aminotetrazole and triethylamine are then added to this mixture, followed by the addition of acetonitrile to give **9.**

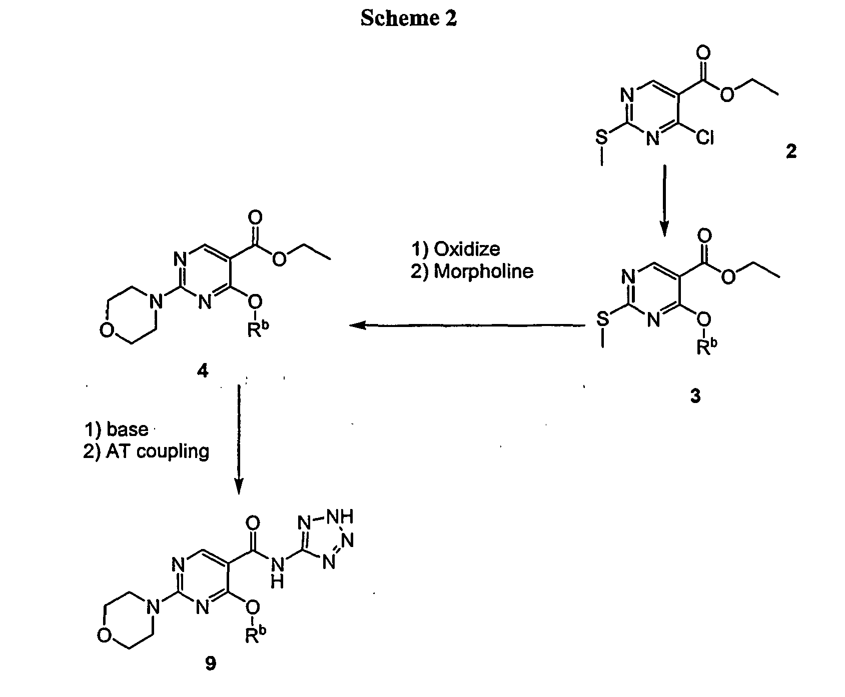

In Scheme **2, 2** is reacted with a R^{b}-OH compound that has been treated with an alkyl lithium reagent such as n-butyl lithium or a hydride base (e.g., NaH) in anhydrous THF to produce **3.** 3 is then oxidized and reacted with morpholine as in Scheme 1 to yield 4. **4** in turn is reacted with aminotetrazole as in Scheme 1 to provide 9.

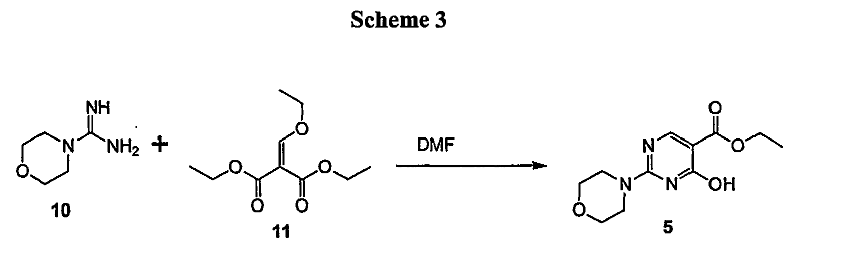

In Scheme **3, 10** (morpholine-4-carboxamidine) is reacted with **11** (2-ethoxymethylene-malonic acid diethyl ester) and sodium acetate in DMF to provide 5 (4-hydroxy-2-morpholin-4-yl-pyrimidine-5-carboxylic acid ethyl ester).

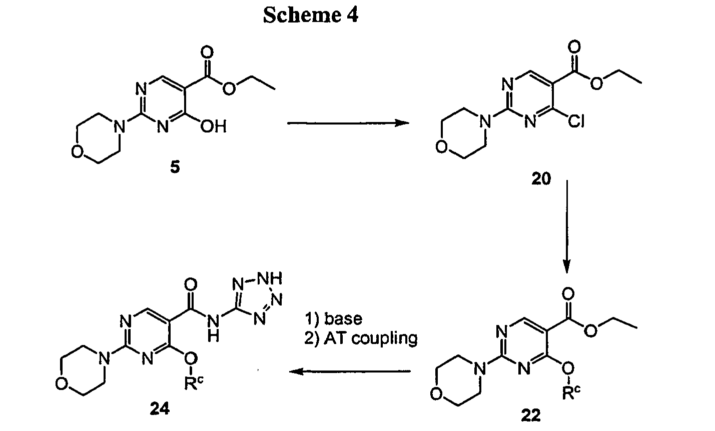

In Scheme 4, 5 in dichloromethane is reacted with oxalyl chloride and a catalytic amount of DMF (dimethylformamide) to yield **20** (e.g., 4-Chloro-2-morpholin-4-yl-pyrimidine-5-carboxylic acid ethyl ester). **20** is reacted with a hydride base (e.g., NaH) treated alcohol (R^{c}-OH) or thiol (R⁴-SH) to provide **22** (e.g., 2-morpholin-4-yl-4-phenoxy-pyrimidine-5-carboxylic acid ethyl ester). R^{c}-OH is a compound that includes but is not limited to a compound of formula HO-L-R^{d} (where L is present, and R^{d} is an optionally substituted phenyl or naphthyl group). Examples of R^{c}-OH include, but are not limited to, 4-cyclohexylphenol and 4-isopropylphenol. **22** is then treated with base and reacted with amino-tetrazole as in Scheme 1 to provide **24.**

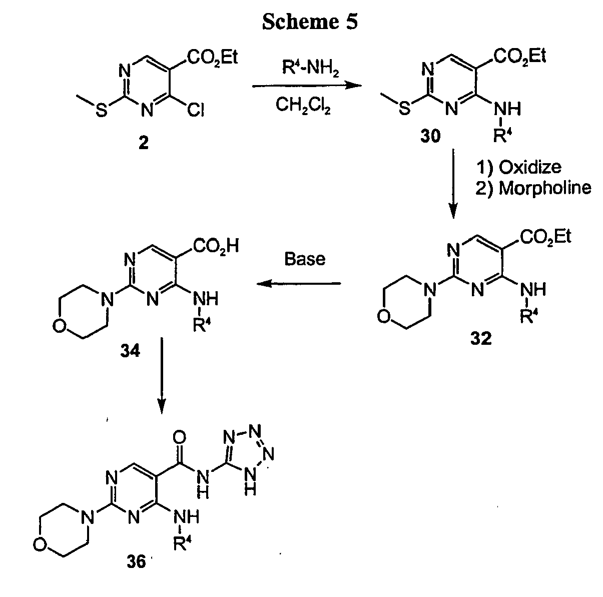

In Scheme 5, 2 in dichloromethane or methylene chloride is reacted with a basic amine such as triethylamine followed by R⁴-NH₂ (e.g., cyclopentylamine, benzyl amine, cyclohexylamine, etc.) to yield **30** (e.g., 4-Cyclopentylamino-2-methylsulfanyl-pyrimidine-5-carboxylic acid ethyl ester). **30** is then oxidized and reacted with morpholine as in Scheme 1 to generate **32** (e.g., 4-cyclopentylamino-2-morpholin-4-yl-pyrimidine-5-carboxylic acid ethyl ester). **32** is then saponified to produce **34** (e.g., 4-cyclopentylamino-2-morpholin-4-yl-pyrimidine-5-carboxylic acid), which is then coupled to 5-amino-tetrazole as described in Scheme 1 to give **36** (e.g., 4-cyclopentylamino-2-morpholin-4-yl-pyrimidine-5-carboxylic acid (1H-tetrazol-5-yl)-amide).

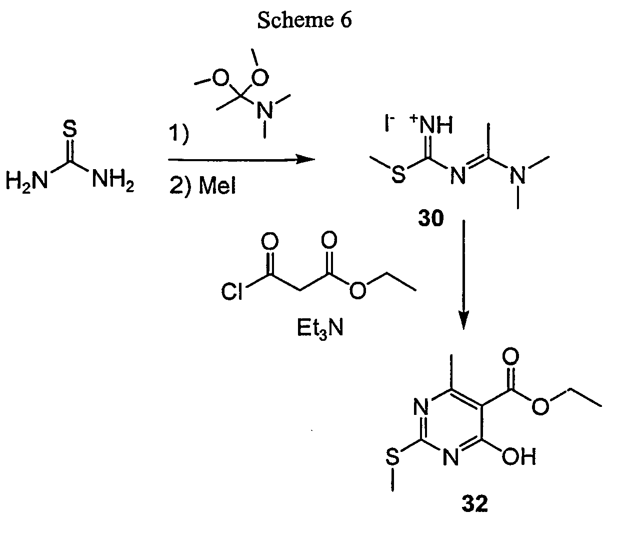

In Scheme 6, thiourea in anhydrous dichloromethane is reacted with N,N-dimethyl acetamide dimethyl acetal under nitrogen gas. The product of the reaction is worked up, resuspended in anhydrous THF and reacted with MeI under nitrogen gas to provide **30** (1-[1-(dimethylamino)ethylidene]-2-methylisothiourea hydroiodide). **30** is then reacted under nitrogen gas with malonyl chloride in anhydrous dichloromethane or chloroform, with the subsequent addition of a base such as triethylamine to provide **32** (ethyl-4-hydroxy-6-methyl-2-(methylthio)-5-pyrimidinecarboxylate). Syntheses using **32** as a starting material can be carried out in an analogous manner to those described above in Schemes 1-5 to provide for compounds of the present invention where R⁶ is a methyl.

### III. EVALUATION OF COMPOUNDS

Compounds of the present invention (e.g., compounds of Formulas I-V and pharmaceutically acceptable salts thereof) can be assayed for their ability to inhibit a PI3K. Examples of these assays are set out below and include in vitro and in vivo assays of PI3K activity.

In certain embodiments of the present invention are compounds that selectively inhibit one or more PI3Ks as compared to one or more enzymes including, but not limited to, a cyclic nucleotide dependent protein kinase, PDGF, a tyrosine kinase, a MAP kinase, a MAP kinase kinase, a MEKK, a cyclin-dependent protein kinase. In other embodiments of the invention are compounds that selectively inhibit one PI3K as compared to another PI3K. For example, in certain embodiments, compounds of the present invention display the ability to selectively inhibit PI3Kγ as compared to PI3Kα or PI3Kβ. A compound selectively inhibits a first enzyme as compared to a second enzyme, when the IC₅₀ of the compound towards the first enzyme is less than the IC₅₀ of the compound towards the second compound. The IC₅₀ can be measured, for example, in an in vitro PI3K assay.

In presently preferred embodiments, compounds of the present invention can be assessed for their ability to inhibit PI3K activity in an in vitro or an in vivo assay (see below).

PI3K assays are carried out in the presence or absence of a PI3K inhibitory compound, and the amount of enzyme activity is compared for a determination of inhibitory activity of the PI3K inhibitory compound.

Samples that do not contain a PI3K inhibitory compound are assigned a relative PI3K activity value of 100. Inhibition of PI3K activity is achieved when the PI3K activity in the presence of a PI3K inhibitory compound is less than the control sample (i.e., no inhibitory compound). The IC₅₀ of a compound is the concentration of compound that exhibits 50% of the control sample activity. In certain embodiments, compounds of the present invention have an IC₅₀ of less than about 100 µM. In other embodiments, compounds of the present invention have an IC₅₀ of about 1 µM or less. In still other embodiments, compounds of the present invention have an IC₅₀ of about 200 nM or less.

PI3Kγ assays have been described in the art (see e.g., Leopoldt et al. J. Biol. Chem., 1998; 273: 7024-7029). Typically, a sample containing a complex of p101 and p110γ protein are combined with Gβ and Gγ proteins (e.g., G protein β₁/γ₂ subunits). Radiolabeled ATP (e.g., γ-³²P-ATP) is then added to this mixture. The lipid substrates are formed by creating PIP₂ containing lipid micelles. The reactions are then started by adding the lipid and enzyme mixtures and are stopped with the addition of H₃PO₄. The lipid products are then transferred to a glass fiber filter plate, and washed with H₃PO₄ several times. The presence of radioactive lipid product (PIP₃) can be measured using radiometric methods that are well-known in the art.

The activity of growth factor regulated PI3Ks can also be measured using a lipid kinase assay. For example, PI3Kα can be assayed using samples that contain a regulatory and a catalytic subunit. An activating peptide (e.g., pY peptide, SynPep Corp.) is added to the sample with radiolabeled ATP. PIP₂ containing lipid micelles are then added to the sample to start the reaction. The reactions are worked up and analyzed as described for the PI3Kγ assay just described. Assays can also be carried out using cellular extracts (Susa et al. J. Biol. Chem., 1992;267:22951-22956).

### IV. IN VIVO EVALUATION OF COMPOUNDS

Compounds of the present invention (e.g., compounds of Formulas I-V and pharmaceutically acceptable salts thereof) can be assayed for their ability to decrease quantitative or qualitative markers of processes such as inflammation. Examples of animal models of arthritis are described below that may be used to assay the ability of a compound of the present invention to treat rheumatoid arthritis.

### Streptococcal cell wall (SCW) induced arthritis assay

Arthritis is induced as described by Schwab, et al., (1991) Infection and Immunity 59:4436-4442 with minor modifications. Rats receive 6 µg sonicated SCW (in 10 µl Dulbecco's PBS (DPBS)) by an intraarticular injection into the right tibiotalar joint on day 0. On day 21, a delayed-type hypersensitivity reaction by systemic SCW can be initiated with 100 µg of SCW (250 µl) administered i.v. For oral compound studies, compounds can be suspended in an appropriate vehicle (e.g., 0.5% hydroxypropyl-methylcellulose/0.2% Tween 80), sonicated, and administered twice daily (10 ml/kg volume) beginning 1 hour prior to initiation of the delayed-type hypersensitivity reaction by i.v. injection of SCW. Compounds are typically administered in amounts between 10 and 500 mg/kg body weight/day, such as 20, 30, 60, 100, 200, and 300 mg/kg/day. Edema measurements are obtained by determining the baseline volumes of the sensitized hindpaw before reactivation on day 21, and comparing them with volumes at subsequent time points such as day 22, 23, 24, and 25. Mercury displacement plethysmography is one method that can be used to assay the paw volume of an animal.

To measure pain, rats are placed in a device that measures the amount of pressure placed on each hind paw. The average difference between the arthritic and normal paws is one method used to measure pain. In addition, inflammatory cytokine levels can be measured in material that has been lavaged from the arthritic joints of an animal and compared to the levels of a non-arthritic joint or typical non-arthritic joint levels.

### Collagen-induced arthritis assay

Type II collagen-induced arthritis (CIA) in mice is an experimental model of rheumatoid arthritis (see e.g., Stuart et al. (1984) Annual Rev. Immunol. 2: 199-218; Wooley (1988) Meth. Enzymol. 162: 361-373). The disease is typically induced by immunization of DBA/1 mice with 100 µg of type II collagen ("CII") (e.g., bovine or chick type II collagen), delivered intradermally at the base of the tail in Freund's complete adjuvant.

A progressive and inflammatory arthritis develops in the majority of mice immunized, characterized by paw width increases of up to 100%. A test compound can be administered to mice in a range of amounts, such as 20, 60, 100, and 200 mg/kg body weight/day. The duration of the test can be several weeks to a few months, such as 40, 60, or 80 days. A clinical scoring index can be used to assess disease progression from erythema and edema (stage 1), joint distortion (stage 2), to joint ankylosis (stage 3). The disease can affect one or all paws in an animal, resulting in a total possible score of 12 for each mouse. Histopathology of an arthritic joint generally reveals synovitis, pannus formation, and cartilage and bone erosions. All mouse strains that are susceptible to CIA are high antibody responders to type II collagen, and there is a marked cellular response to CII.

Dosing of a test compound is usually performed either prophylactically (for 10 weeks) or therapeutically (for 10 days when disease is first observed). Mice are typically examined daily for the development of arthritis and a clinical score is often assigned. Serum can also retrieved from each animal at various time points for measurement of antibodies or cytokines (as required). At the end of the study the mice can be euthanized and a quantitative histopathology score assigned.

### Monoclonal Antibody-Induced Arthritis assay

Another experimental model of rheumatoid arthritis involves injecting antibodies to type II collagen epitopes into a mouse to induce arthritis in a few days (see e.g., Burkhardt et al. (2002) Arthritis & Rheumatism 46: 2339-2348; Terato et la. (1992) J. Immunol. 148: 2103-2108). Cocktails of four antibodies are commercially available for use in this model (Arthrogen-CIA® Monoclonal Antibody Cocktail, CHEMICON International, Inc., Temecula, CA). This model does not require DBA/1 strain mice. A test compound can be administered to mice one or more times in a range of amounts, such as 20, 60, 100, and 200 mg/kg body weight/day at any time during the study. Ankle and paw swelling are typically measured quantitatively or qualitatively as endpoints as well as histology. In addition, serum can be retrieved from each animal at various time points for measurement of antibodies or cytokines.

### V. PHARMACEUTICALLY ACCEPTABLE SALTS AND SOLVATES

The compounds to be used in the present invention can exist in unsolvated forms as well as solvated forms, including hydrated forms. In general, the solvated forms, including hydrated forms, are equivalent to unsolvated forms and are intended to be encompassed within the scope of the present invention.

The compounds of the present invention (e.g., compounds of Formulas I-V) are capable of further forming both pharmaceutically acceptable salts, including but not limited to acid addition and/or base salts. Pharmaceutically acceptable salts of the compounds of formula (I) include the acid addition and base salts (including disalts) thereof. Examples of suitable salts can be found for example in Stahl and Wermuth, Handbook of Pharmaceutical Salts: Properties, Selection, and Use, Wiley-VCH, Weinheim, Germany (2002); and Berge et al., "Pharmaceutical Salts," J. of Pharmaceutical Science, 1977;66:1-19.

Pharmaceutically acceptable acid addition salts of the compounds of Formulas I-V include non-toxic salts derived from inorganic acids such as hydrochloric, nitric, phosphoric, sulfuric, hydrobromic, hydriodic, phosphorus, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include the acetate, aspartate, benzoate, besylate (benzenesulfonate), bicarbonate/carbonate, bisulfate, caprylate, camsylate (camphor sulfonate), chlorobenzoate, citrate, edisylate (1,2-ethane disulfonate), dihydrogenphosphate, dinitrobenzoate, esylate (ethane sulfonate), fumarate, gluceptate, gluconate, glucuronate, hibenzate, hydrochloride/chloride, hydrobromide/bromide, hydroiodide/iodide, isobutyrate, monohydrogen phosphate, isethionate, D-lactate, L-lactate, malate, maleate, malonate, mandelate, mesylate (methanesulfonate), metaphosphate, methylbenzoate, methylsulfate, 2-napsylate (2-naphthalene sulfonate), nicotinate, nitrate, orotate, oxalate, palmoate, phenylacetate, phosphate, phthalate, propionate, pyrophosphate, pyrosulfate, saccharate, sebacate, stearate, suberate, succinate sulfate, sulfite, D-tartrate, L-tartrate, tosylate (toluene sulfonate), and xinafoate salts, and the like of compounds of Formulas I-V. Also contemplated are the salts of amino acids such as arginate, gluconate, galacturonate, and the like.

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metal hydroxides, or of organic amines. Examples of metals used as cations are aluminum, calcium, magnesium, potassium, sodium, and the like. Examples of suitable amines include arginine, choline, chloroprocaine, N,N'-dibenzylethylenediamine, diethylamine, diethanolamine, diolamine, ethylenediamine (ethane-1,2-diamine), glycine, lysine, meglumine, N-methylglucamine, olamine, procaine (benzathine), and tromethamine.

The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

### VI. PHARMACEUTICAL COMPOSITIONS AND METHODS OF ADMINISTRATION

This invention also provides for pharmaceutical compositions comprising a therapeutically effective amount of a compound of Formulas I-V, or a pharmaceutically acceptable salt thereof together with a pharmaceutically acceptable carrier, diluent, or excipient therefor. The phrase "pharmaceutical composition" refers to a composition suitable for administration in medical or veterinary use. The phrase "therapeutically effective amount" means an amount of a compound, or a pharmaceutically acceptable salt thereof, sufficient to inhibit, halt, or allow an improvement in the disorder or condition being treated when administered alone or in conjunction with another pharmaceutical agent or treatment in a particular subject or subject population. For example in a human or other mammal, a therapeutically effective amount can be determined experimentally in a laboratory or clinical setting, or may be the amount required by the guidelines of the United States Food and Drug Administration, or equivalent foreign agency, for the particular disease and subject being treated.

It should be appreciated that determination of proper dosage forms, dosage amounts, and routes of administration is within the level of ordinary skill in the pharmaceutical and medical arts, and is described below.

A compound of the present invention can be formulated as a pharmaceutical composition in the form of a syrup, an elixir, a suspension, a powder, a granule, a tablet, a capsule, a lozenge, a troche, an aqueous solution, a cream, an ointment, a lotion, a gel, an emulsion, etc. Preferably, a compound of the present invention will cause a decrease in symptoms or a disease indicia associated with a PI3K-mediated disorder as measured quantitatively or qualitatively.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired.

The powders and tablets contain from 1% to 95% (w/w) of the active compound. In certain embodiments, the active compound ranges from 5% to 70% (w/w). Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 1000 mg, preferably 1.0 mg to 100 mg, or from 1% to 95% (w/w) of a unit dose, according to the particular application and the potency of the active component. The composition can, if desired, also contain other compatible therapeutic agents.

Pharmaceutically acceptable carriers are determined in part by the particular composition being administered, as well as by the particular method used to administer the composition. Accordingly, there are a wide variety of suitable formulations of pharmaceutical compositions of the present invention (see, e.g., Remington: The Science and Practice of Pharmacy, 20th ed., Gennaro et al. Eds., Lippincott Williams and Wilkins, 2000).

A compound of the present invention, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane nitrogen, and the like.

Formulations suitable for parenteral administration, such as, for example, by intravenous, intramuscular, intradermal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and nonaqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. The formulations of compounds can be presented in unit-dose or multi-dose sealed containers, such as ampules and vials. Injection solutions and suspensions can be prepared from sterile powders, granules, and tablets of the kind previously described.

The dose administered to a subject, in the context of the present invention should be sufficient to affect a beneficial therapeutic response in the subject over time. The term "subject" refers to a member of the class Mammalia. Examples of mammals include, without limitation, humans, primates, chimpanzees, rodents, mice, rats, rabbits, horses, livestock, dogs, cats, sheep, and cows.

The dose will be determined by the efficacy of the particular compound employed and the condition of the subject, as well as the body weight or surface area of the subject to be treated. The size of the dose also will be determined by the existence, nature, and extent of any adverse side-effects that accompany the administration of a particular compound in a particular subject. In determining the effective amount of the compound to be administered in the treatment or prophylaxis of the disorder being treated, the physician can evaluate factors such as the circulating plasma levels of the compound, compound toxicities, and/or the progression of the disease, etc. In general, the dose equivalent of a compound is from about 1 µg/kg to 100 mg/kg for a typical subject. Many different administration methods are known to those of skill in the art.

For administration, compounds of the present invention can be administered at a rate determined by factors that can include, but are not limited to, the LD₅₀ of the compound, the pharmacokinetic profile of the compound, contraindicated drugs, and the side-effects of the compound at various concentrations, as applied to the mass and overall health of the subject. Administration can be accomplished via single or divided doses.

Examples of a typical tablet, parenteral, and patch formulation include the following:

**TABLET FORMULATION EXAMPLE 1**

| Tablet Formulation | |
|---|---|
| Ingredient | Amount |
| Compound of Formulas I-V | 50 mg |
| Lactose | 80 mg |
| Cornstarch (for mix) | 10 mg |
| Cornstarch (for paste) | 8 mg |
| Magnesium Stearate (1%) | 2 mg |
| | 150 mg |

The compounds of the present invention (e.g., a compound of Formulas I-V, or a pharmaceutically acceptable salt thereof) can be mixed with the lactose and cornstarch (for mix) and blended to uniformity to a powder. The cornstarch (for paste) is suspended in 6 mL of water and heated with stirring to form a paste. The paste is added to the mixed powder, and the mixture is granulated. The wet granules are passed through a No. 8 hard screen and dried at 50°C. The mixture is lubricated with 1% magnesium stearate and compressed into a tablet. The tablets are administered to a patient at the rate of 1 to 4 each day for treatment of a PI3K-mediated disorder or condition.

### PARENTERAL SOLUTION FORMULATION EXAMPLE 1

In a solution of 700 mL of propylene glycol and 200 mL of water for injection can be added 20.0 g of a compound of the present invention. The mixture is stirred, and the pH is adjusted to 5.5 with hydrochloric acid. The volume is adjusted to 1000 mL with water for injection. The solution is sterilized, filled into 5.0 mL ampules, each containing 2.0 mL (40 mg of invention compound), and sealed under nitrogen. The solution is administered by injection to a subject suffering from a PI3K-mediated disorder or condition and in need of treatment.

### PATCH FORMULATION EXAMPLE 1

Ten milligrams of a compound of the present invention can be mixed with 1 mL of propylene glycol and 2 mg of acrylic-based polymer adhesive containing a resinous cross-linking agent. The mixture is applied to an impermeable backing (30 cm²) and applied to the upper back of a patient for sustained release treatment of a PI3K-mediated disorder or condition.

### VH. METHODS FOR TREATING PI3K-MEDIATED DISORDERS AND CONDITIONS

The compounds of the present invention and pharmaceutical compositions comprising a compound of the present invention can be administered to a subject suffering from a PI3K-mediated disorder or condition. PI3K-mediated disorders and conditions can be treated prophylactically, acutely, and chronically using compounds of the present invention, depending on the nature of the disorder or condition. Typically, the host or subject in each of these methods is human, although other mammals can also benefit from the administration of a compound of the present invention.

In therapeutic applications, the compounds of the present invention can be prepared and administered in a wide variety of oral and parenteral dosage forms. The term "administering" refers to the method of contacting a compound with a subject. Thus, the compounds of the present invention can be administered by injection, that is, intravenously, intramuscularly, intracutaneously, subcutaneously, intraduodenally, parentally, or intraperitoneally. Also, the compounds described herein can be administered by inhalation, for example, intranasally. Additionally, the compounds of the present invention can be administered transdermally, topically, via implantation, transdermally, topically, and via implantation. In certain embodiments, the compounds of the present invention are delivered orally. The compounds can also be delivered rectally, bucally, intravaginally, ocularly, andially, or by insufflation.

The compounds utilized in the pharmaceutical method of the invention can be administered at the initial dosage of about 0.001 mg/kg to about 100 mg/kg daily. In certain embodiments, the daily dose range is from about 0.1 mg/kg to about 10 mg/kg. The dosages, however, may be varied depending upon the requirements of the subject, the severity of the condition being treated, and the compound being employed. Determination of the proper dosage for a particular situation is within the skill of the practitioner. Generally, treatment is initiated with smaller dosages, which are less than the optimum dose of the compound. Thereafter, the dosage is increased by small increments until the optimum effect under circumstances is reached. For convenience, the total daily dosage may be divided and administered in portions during the day, if desired. The term "treatment" includes the acute, chronic, or prophylactic diminishment or alleviation of at least one symptom or characteristic associated with or caused by the disorder being treated. For example, treatment can include diminishment of several symptoms of a disorder, inhibition of the pathological progression of a disorder, or complete eradication of a disorder. The compounds of the present invention can be co-administered to a subject. The term "co-administered" means the administration of two or more different pharmaceutical agents or treatments (e.g., radiation treatment) that are administered to a subject by combination in the same pharmaceutical composition or separate pharmaceutical compositions. Thus co-administration involves administration at the same time of a single pharmaceutical composition comprising two or more pharmaceutical agents or administration of two or more different compositions to the same subject at the same or different times. For example, a subject that is administered a first dosage that comprises a compound of the present invention at 8 a.m. and then is administered CELEBREX® at 1-12 hours later, e.g., 6 p.m., of that same day has been co-administered with a compound of the present invention and CELEBREX®. Alternatively, for example, a subject could be administered with a single dosage comprising a compound of the present invention and CELEBREX ® at 8 a.m. has been co-administered with a compound of the present invention and CELEBREX®.

Thus, compounds of the invention can also be co-administered with compounds that are useful for the treatment of cancer (e.g., cytotoxic drugs such as TAXOL®, taxotere, GLEEVEC® (Imatinib Mesylate), adriamycin, daunomycin, cisplatin, etoposide, a vinca alkaloid, vinblastine, vincristine, methotrexate, or adriamycin, daunomycin, cis-platinum, etoposide, and alkaloids, such as vincristine, farnesyl transferase inhibitors, endostatin and angiostatin, VEGF inhibitors, and antimetabolites such as methotrexate. The compounds of the present invention may also be used in combination with a taxane derivative, a platinum coordination complex, a nucleoside analog, an anthracycline, a topoisomerase inhibitor, or an aromatase inhibitor). Radiation treatments can also be co-administered with a compound of the present invention for the treatment of cancers.

The compounds of the invention can also be co-administered with compounds that are useful for the treatment of a thrombolytic disease, heart disease, stroke, etc., (e.g., aspirin, streptokinase, tissue plasminogen activator, urokinase, anticoagulants, antiplatelet drugs (e.g., PLAVIX®; clopidogrel bisulfate), a statin (e.g., LIPITOR® (Atorvastatin calcium), ZOCOR® (Simvastatin), CRESTOR® (Rosuvastatin), etc.), a Beta blocker (e.g, Atenolol), NORVASC® (amlodipine besylate), and an ACE inhibitor (e.g., Accupril® (Quinapril Hydrochloride), Lisinopril, etc.).

The compounds of the invention can also be co-administered for the treatment of hypertension with compounds such as ACE inhibitors, lipid lowering agents such as statins, LIPITOR® (Atorvastatin calcium), calcium channel blockers such as NORVASC® (amlodipine besylate). The compounds of the present invention may also be used in combination with fibrates, beta-blockers, NEPI inhibitors, Angiotensin-2 receptor antagonists and platelet aggregation inhibitors.

For the treatment of inflammatory diseases, including rheumatoid arthritis, the compounds of the invention may be co-administered with agents such as TNF-α inhibitors such as anti-TNFα monoclonal antibodies (such as REMICADE®, CDP-870 and HUMIRA^{™} (adalimumab) and TNF receptor-immunoglobulin fusion molecules (such as ENBREL®), IL-1 inhibitors, receptor antagonists or soluble IL-1Rα (e.g. KINERET^{™} or ICE inhibitors), nonsteroidal anti-inflammatory agents (NSAIDS), piroxicam, diclofenac, naproxen, flurbiprofen, fenoprofen, ketoprofen ibuprofen, fenamates, mefenamic acid, indomethacin, sulindac, apazone, pyrazolones, phenylbutazone, aspirin,COX-2 inhibitors (such as CELEBREX® (celecoxib), VIOXX® (rofecoxib), BEXTRA® (valdecoxib) and etoricoxib, metalloprotease inhibitors (preferably MMP-13 selective inhibitors), NEUROTIN®, pregabalin, sulfasalazine, low dose methotrexate, leflunomide, hydroxychloroquine, d-penicillamine, auranofin or parenteral or oral gold.

The compounds of the invention may be co-administered with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flurbiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics and intraarticular therapies such as corticosteroids and hyaluronic acids such as hyalgan and synvisc.

The compounds of the invention may also be co-administered with antiviral agents such as Viracept, AZT, aciclovir and famciclovir, and antisepsis compounds such as Valant.

The compounds of the present invention may further be co-administered with CNS agents such as antidepressants (such as sertraline), anti-Parkinsonian drugs (such as deprenyl, L-Dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), NEURONTIN®, pregabalin, and anti-Alzheimer's drugs such as ARICEPT®, tacrine, propentofylline or metrifonate.

The compounds of the present invention may additionally be co-administered with osteoporosis agents such as EVISTA® (raloxifene hydrochloride), droloxifene, lasofoxifene or FOSAMAX® and immunosuppressant agents such as FK-506 and rapamycin.

### EXAMPLES

| 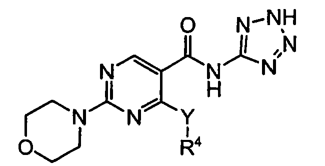 | | | | |
|---|---|---|---|---|
| **Ex.** | -Y-R⁴ | **MS^{a}** | **NMR^{b} and/or combustion analysis** | **IC⁵⁰ (µM)** |
| 1 | 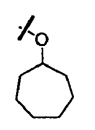 | 389.3 | 1.50 (m, 6H), 1.65 (m, 2H), 1.85 (m, 2H), 2.01 (m, 2H), 3.65 (m, 4H), 3.78 (m, 4H) 5.39 (m, 1H), 8.67 (s, 1H), 10.88 (s, 1H), 16.00 (s, 1H). | 0.197 |
| | | | Calculated C-52.57%, H-6.23%, N-28.85%, Cl-0%, H₂O-0%. Found C-52.36%, H-6.24%, N-28.72%, Cl-0%, H₂O-0%. | |
| 2 | 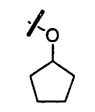 | 361.2 | (CDCl₃) 1.78 (m,3H), 1.7-2.2 (m, 5H), 3.78 (m, 4H), 3.95 (m, 4H), 5.69 (m, 1H), 8.97 (s, 1H), 10.46 (s, 1H), 13.29 (s, 1H). | 1.541 |
| | | | Calculated C-49.99%, H-5.59%, N-31.09%. Found C-49.97%, H-5.54%, N-30.86%. | |
| 3 | 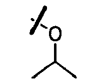 | 335.2 | 1.38 (d, J=6.34Hz, 6H), 3.65 (m, 4H), 3.80 (m, 4H), 5.40 (m, 1H), 8.66 (s, 1H), 11.01 (s, 1H), 16.01 (s, 1H). | 87.4965 |
| 4 | 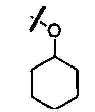 | 375.3 | 1.44 (m, 4H), 1.66 (m, 4H), 3.65 (m, 4H), 3.78 (m, 4H), 5.27 (m, 1H), 8.69 (s, 1H), 10.92 (s, 1H), 16.02 (s, 1H) | 1.735 |
| 5 | 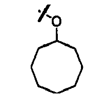 | 403.3 | 1.53-1.96 (m, 14H), 3.66 (m, 4H), 3.80 (m, 4H), 5.41 (m, 1H), 8.68 (s, 1H), 10.88 (s, 1H), 16.03 (s, 1H). | 0.5025 |
| 6 | 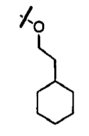 | 403.2 | 0.91-1.73 (m, 13H), 3.65 (m, 4H), 3.80 (m, 4H), 4.47 (m, 2H), 8.65 (s, 1H), 10.96 (s, 1H). | 10.07 |
| | | | Calculated C-53.72%, H-6.51%, N-27.84%. Found C-53.56%, H-6.60%, N-27.64%. | |
| 7 | 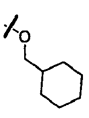 | 389.2 | 1.05-1.79 (m, 11H), 3.64 (m, 4H), 3.80 (m, 4H), 4.27 (d, J=5.01Hz, 2H), 8.66 (s, 1H), 11.02 (s, 1H), 16.02 (s, 1H). | 1.835 |
| | | | Calculated C-52.57%, H-6.23%, N-28.85%. Found C-52.21%, H-6.19%, N-29.02%. | |
| 8 | 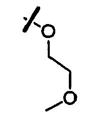 | 351.2 | 3.32 (s, 3H), 3.65 (m, 4H), 3.71 (m, 2H), 3.81 (m, 4H), 4.56 (m, 2H), 8.69 (s, 1H), 10.95 (s, 1H). Calculated C-44.57%, H-5.18%, N-31.98%. Found C-44.81%, H-5.02%, N-31.60%. | 2.32 |
| 9 | 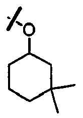 | 403.3 | 0.94 (d, *J*=7.81 Hz, 6H), 1.30 (m, 3H), 1.46 (m, 2H), 1.67 (m, 1H), 1.87 (m, 1H), 2.05 (m, 1H), 3.66 (m, 4H), 3.79 (m, 4H), 5.30 (m, 1H), 8.66 (s, 1H), 10.96 (s, 1H), 15.97 (s, 1H). | 1.36 |
| 10 | 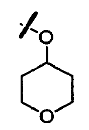 | 377.3 | 1.76 (m, 2H), 2.04 (m, 2H), 3.55 (m, 2H), 3.65 (m, 4H), 3.78 (m, 4H), 3.82 (m, 2H), 5.40 (m, 1H), 8.67 (s, 1H), 11.06 (s, 1H), 16.01 (s, 1H). Calculated C-47.87%, H-5.36%, N-29.77%. Found C-47.74%, H-5.34%, N-29.85%. | 10.48 |
| 11 | 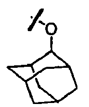 | 427.2 | 1.59-2.21 (m, 14H), 3.64 (m, 4H), 3.77 (m, 4H), 5.38 (m, 1H), 8.70 (s, 1H), 10.95 (s, 1H). | 0.485 |
| 12 | 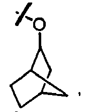 | 387.2 | 1.08-2.22 (m, 10H), 3.64 (m, 4H), 3.78 (m, 4H), 5.28 (m, 1H), 8.65 (s, 1H), 10.98 (s, 1H). | 0.845 |
| 13 | 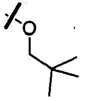 | 363.2 | 1.01 (s, 9H), 3.65 (m, 4H), 3.80 (m, 4H), 4.15 (s, 2H), 8.67 (s, 1H), 10.96 (s, 1H), 16.03 (s, 1H). | 2.06 |
| 14 | 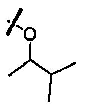 | 363.2 | 0.95 (d, J=6.83Hz, 3H), 0.97 (d, J=6.83Hz, 3H), 1.30 (d, J=6.34 Hz, 3H), 1.98 (m, 1H), 3.66 (m, 4H), 3.79 (m, 4H), 5.22 (m, 1H), 8.69 (s, 1H), 10.91 (s, 1H), 16.01 (s, 1H). | 1.16 |
| 15 | 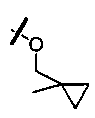 | 361.2 | 0.41 (m,2H), 0.59 (m, 2H), 1.18 (s, 3H), 3.62-3.65 (m, 4H), 3.78-3.82 (m, 4H), 4.27 (s, 2H), 8.68 (s, 1H), 10.98 (s, 1H), 16.02 (s, 1H). Calculated C-49.99%, H-5.59%, N-31.09%. Found C-49.94%, H-5.50%, N-31.26%. | 1.69 |
| 16 | 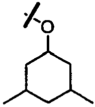 | 403.3 | 0.60 (m, 1H), 0.92 (d, *J*=6.34 Hz, 6H), 1.06 (m, 2H), 1.60 (m, 3H), 2.12 (m, 2H), 3.66 (m, 4H), 3.79 (m, 4H), 5.17 (m, 1H), 8.67 (m, 1H), 10.93 (s, 1H). | 0.21 |

| | | | | |
|---|---|---|---|---|
| ^{a} MS: M+1 APCI unless otherwise indicated. ^{b}DMSO, 400 MHz, δ values reported. | | | | |

**Intermediate 1. 2-methylsulfanyl-4-(4-nitro-benzyloxy)-pyrimidine-5-carboxylic acid ethyl ester.** To a stirring solution of 4-nitrobenzyl alcohol (16.7 g, 109 mmol) in anhydrous THF (120 mL) at -78 °C, was added *n*BuLi (1.6M in hexanes, 75 mL, 120 mmol) via syringe. The reaction was stirred at -78 °C for ten minutes and then the cold bath was removed and the mix was allowed to warm to room temperature. The nitrobenzyl alkoxide solution was poured into a stirring solution of ethyl 4-chloro-2-methylthio-5-pyrimidine carboxylate (25.0 g, 107.3 mmol) in anhydrous THF (120 mL). The reaction was stirred at room temperature for 15 minutes. The mix was diluted with H₂O and was extracted several times with EtOAc. The organic extracts were washed with brine, dried over Na₂SO₄, decanted, and concentrated to give a solid. The solid was slurried in a minimum of dichloromethane and filtered. The solid was collected and dried overnight in the vacuum oven to give the desired title product (19.4 g, 51.8% yield), as a solid.
MS: M+1 = 350.1.

**Intermediate 2. 2-methanesulfinyl-4-(4-nitro-benzyloxy)-pyrimidine-5-carboxylic acid ethyl ester.** To a stirring solution of **Intermediate 1** (19.4 g, 55.6 mmol) in chloroform (185 mL) was added *m*CPBA (13.8 g, 61.2 mmol) portionwise. The exothermic reaction was moderated with an ice-water bath to keep the reaction temperature near room temperature. The reaction was then stirred at room temperature for 25 minutes. The mix was quenched with saturated aqueous NaHCO₃ and diluted with water. The dilution was extracted several times with EtOAc. The organic extracts were washed with brine, dried over Na₂SO₄, filtered through celite, and concentrated to give the desired title product (18.84 g, 92.8% yield), as an orange solid. The product was carried on without further purification. MS: M+1 = 366.1 (APCI).

**Intermediate 3. 2-morpholin-4-yl-4-(4-nitro-benzyloxy)-pyrimidine-5-carboxylic acid ethyl ester. Intermediate 2** (35.4 g, 97.0 mmol) was stirred in morpholine (16.9 mL) at 100 °C for fifteen minutes. The reaction was allowed to cool to room temperature and then was diluted with toluene (~100 mL). The dilution mixture was concentrated *en vacuo.* The remaining residue was boiled in EtOAc (~300 mL) and then filtered. The liquors were concentrated *en vacuo.* The remaining solid was slurried in a minimum of dichloromethane and filtered. The solid was collected and dried overnight in a vacuum oven to give the desired title product (12.8 g, 34.0% yield), as a white solid. MS: M+1 = 389.1 (APCI). ¹H-NMR (400 MHz, CDCl₃) 8.79 (s, 1H), 8.24 (m, 2H), 7.68 (m, 2H), 5.54 (s, 2H), 4.33 (q, J=7.2Hz, 2H), 3.99 (m, 2H), 3.85 (m, 2H), 3.74 (m, 2H), 3.23 (m, 2H), 1.36 (t, J=7.2Hz, 3H).

**Intermediate 4. 4-hydroxy-2-morpholin-4-yl-pyrimidine-5-carboxylic acid ethyl ester. Intermediate 3** (4.00 g, 10.3 mmol) was stirred with Pd/C 20% (0.400 g) in ethanol (52 mL) at room temperature under a hydrogen balloon for 16 hours. An additional portion of Pd/C 20% (0.400 g) was added and hydrogen was bubbled through the reaction mixture for ten minutes. The reaction was stirred under a hydrogen balloon at room temperature for four hours. The mix was filtered through celite. The filter cake was rinsed with EtOAc and dichloromethane. The liquors were concentrated to give a solid. The solid was slurried in a minimum of diethyl ether at room temperature for two hours and then filtered. The solid was collected and dried overnight in a vacuum oven to give the desired title product (2.49 g, 95.6% yield), as an off white solid. MS: M+1 = 254.1 (APCI). ¹H-NMR (400 MHz, CDCl₃) 8.67 (s, 1H), 4.38 (q, J=7.1Hz, 2H), 3.93 (m, 4H), 3.74 (m, 4H), 1.39 (t, J=7.1Hz, 3H).

**Intermediate 5. 4-Cycloheptyloxy-2-morpholin-4-yl-pyrimidine-5-carboxylic** acid ethyl ester. **Intermediate 4** (10.00 g, 39.49 mmol) was combined with cycloheptanol (7.134 mL, 59.23 mmol), DEAD (8.25 g, 47.4 mmol), and triphenyl phosphine (12.4 g, 47.4 mmol) in THF (150 mL). The reaction was stirred at room temperature for 18 hours. HPLC analysis indicated a new product along with excess triphenyl phosphine and triphenylphosphine oxide. The reaction mixture was concentrated *en vacuo.* The concentrate was purified by silica gel chromatography (0-30% EtOAc-hexanes gradient elution, TLC in 20% EtOAc-Hexanes) to give the desired title product (10.84 g, 78.57% yield) as a light pink oil. MS: M+1 = 350.3 (APCI). ¹H-NMR (400 MHz, CDC13) ppm 1.33 (t, *J*=7.20 Hz, 3H), 1.54 (m, 5H), 1.75 (m, 2H), 1.94 (m, 5H), 3.74 (m, 1H), 3.92 (m, 4H), 4.28 (q, *J*=7.08 Hz, 2H), 5.35 (m, 1H), 8.70 (m, 1H).

**Intermediate 6. 4-cycloheptyloxy-2-morpholin-4-yl-pyrimidine-5-carbozylic** acid. **Intermediate 5** (10.84 g, 31.02 mmol) was stirred with dioxane (60 mL) and 1N LiOH (40 mL) in a round bottom flask at 80°C for two hours. The mix was allowed to cool to room temperature and then was slowly and carefully acidified with 1N HCl to a pH of about 1. A white solid precipitated. The mix was diluted with more H₂O and then filtered. The filter cake was rinsed with H₂O, collected, and dried overnight in the vacuum oven to give the desired title product (5.70 g, 57.2% yield) as a light yellow solid. MS: M+1 = 322.2 (APCI). ¹H-NMR (400 MHz, CDCl3) ppm 1.4-1.7 (m, 8H), 1.89 (s, 2H), 2.09 (m, 2H), 3.76 (m, 4H), 3.89 (m, 4H), 5.45 (m, 1H), 8.86 (s, 1H).

**Example 1. 4-Cycloheptyloxy-2-morpholin-4-yl-pyrimidine-5-carboxylic acid (2H-tetrazol-5-yl)-amide. Intermediate 6** (5.70 g, 17.7 mmol) was mixed with - but did not dissolve in anhydrous dichloromethane (50mL). About five drops of DMF were added. Oxalyl chloride (1.70 mL, 19.5 mmol) was added via syringe. The mix bubbled and slowly became homogeneous. The reaction was stirred at room temperature for ten minutes. HPLC analysis of an aliquot quenched into MeOH/K₂CO₃ indicated complete consumption of the starting acid and the formation of a new, less-polar product. 5-Amino tetrazole (3.17 g, 37.2 mmol), triethylamine (5.19 mL, 37.3 mmol), and finally acetonitrile (50mL) were added. The reaction was stirred at 80°C for 60 minutes. HPLC analysis of an aliquot quenched into MeOH/K₂CO₃ indicated a new product. The reaction vessel was opened and allowed to boil nearly dry. The oil bath was removed and the mix was allowed to cool to room temperature. The remaining tar was taken up in a minimum of acetonitrile (~15mL). The mix was diluted with H₂O (~100mL) and acidified with 1N HCl until a fluffy white solid precipitated. The solid was filtered and rinsed with H₂O. The cake was collected and slurried in a minimum of MeOH for 30 minutes. The slurry was filtered and the cake was rinsed with a minimum of MeOH. The solid was dried overnight in a vacuum oven to give the desired title product (4.832 g, 70.14% yield) as small, light yellow needle crystals. Melting Point: 287.1-287.4 °C (determined on Buchi Melting Point B-545 apparatus).

The title compounds of Examples 2-15 were synthesized in a manner analogous to Example 1 by replacing cycloheptanol with an appropriate alcohol (e.g. cyclopentanol).

### Example 2. 4-Cyclopentyloxy-2-morpholin-4-yl-pyrimidine-5-carboxylic acid (2H-tetrazol-5-yl)-amide.

### Example 3. 4-Isopropoxy-2-morpholin-4-yl-pyrimidine-5-carboxylic acid (2H-tetrazol-5-yl)-amide.

### Example 4. 4-Cyclohexyloxy-2-morpholin-4-yl-pyrimidine-5-carboxylic acid (2H-tetrazol-5-yl)-amide.

### Example 5. 4-Cyclooctyloxy-2-morpholin-4-yl-pyrimidine-5-carboxylic acid (2H-tetrazol-5-yl)-amide.

### Example 6.4-(2-Cyclohexyl-ethoxy)-2-morpholin-4-yl-pyrimidine-5-carboxylic acid (2H-tetrazol-5-yl)-amide.

### Example 7.4-Cyclohexylmethoxy-2-morpholin-4-yl-pyrimidine-5-carboxylic acid (2H-tetrazol-5-yl)-amide.

### Example 8. 4-(2-Methoxy-ethoxy)-2-morpholin-4-yl-pyrimidine-5-carboxylic acid (2H-tetrazol-5-yl)-amide.

### Example 9. 4-(3,3-Dimethyl-cyclohexyloxy)-2-morpholin-4-yl-pyrimidine-5-carboxylic acid (2H-tetrazol-5-yl)-amide.

### Example 10. 2-Morpholin-4-yl-4-(tetrahydro-pyran-4-yloxy)-pyrimidine-5-carboxylic acid (2H-tetrazol-5-yl)-amide.

### Example 11. 4-(Adamantan-2-yloxy)-2-morpholin-4-yl-pyrimidine-5-carboxylic acid (2H-tetrazol-5-yl)-amide.

### Example 12. 4-(Bicyclo[2.2.1]hept-2-yloxy)-2-morpholin-4-yl-pyrimidine-5-carboxylic acid (2H-tetrazol-5-yl)-amide.

### Example 13. 4-(2,2-Dimethyl-propoxy)-2-morpholin-4-yl-pyrimidine-5-carboxylic acid (2H-tetrazol-5-yl)-amide.

### Example 14. 4-(1,2-Dimethyl-propoxy)-2-morpholin-4-yl-pyrimicline-5-carboxylic acid (2H-tetrazol-5-yl)-amide.

### Example 15. 4-(1-Methyl-cyclopropylmethoxy)-2-morpholin-4-yl-pyrimidine-5-carboxylic acid (2H-tetrazol-5-yl)-amide.

**Intermediate 7. 4-(3,5-Dimethyl-cyclohexyloxy)-2-methylsulfanyl-pyrimidine-5-carboxylic acid ethyl ester.** To a -78 °C solution of 3,5-dimethylcyclohexanol (6.18 mL, 43.1 mmol) in anhydrous THF (100 mL) was added *n*BuLi (1.6M in hexanes, 26.9 mL, 43.1 mmol) via syringe. The cold bath was removed and the reaction was allowed to warm to room temperature. The reaction mix was then poured into a stirring solution of ethyl 4-chloro-2-methylthio-5-pyrimidine carboxylate (10.0 g, 43.1 mmol) in anhydrous THF (100 mL). The mixture was stirred at room temperature for two hours. The mix was diluted with H₂O and extracted several times with EtOAc. The organic extracts were washed with brine, dried over Na₂SO₄, decanted, and concentrated. The concentrate was purified by silica gel chromatography (0-12% EtOAc-hexanes gradient elution, TLC in 10% EtOAc-Hexanes) to give the desired title product (5.95 g, 42.6% yield) as a colorless oil. MS: M+1 = 325.3 (APCI).

**Intermediate 8. 4-(3,5-Dimethyl-cyclohexyloxy)-2-methanesulfinyl-pyrimidine-5-carboxylic acid ethyl ester.** To a stirring solution of **Intermediate** 8 (5.89 g, 18.2 mmol) in chloroform (60 mL) was added *m*CPBA (4.90 g, 21.8 mmol) portionwise. The exothermic reaction was moderated with an ice-water bath to keep the reaction temperature near room temperature. The reaction was then stirred at room temperature for 25 minutes. The mix was quenched with saturated aqueous NaHCO₃ and diluted with water. The dilution was extracted several times with EtOAc. The organic extracts were washed with brine, dried over Na₂SO₄, filtered through celite, and concentrated to give the desired title product in quantitative yield. The product was carried on without further purification. MS: M+1 = 341.1 (APCI).

**Intermediate 9. 4-(3,5-Dimethyl-cyclohexyloxy)-2-morpholin-4-yl-pyrimidine-5-carboxylic acid ethyl ester. Intermediate 8** (7.13 g, 21.0 mmol) was stirred in morpholine (9.14 mL) at 100 °C for one hour. The reaction was allowed to cool to room temperature. The entire reaction mixture was purified by silica gel chromatography (0-16% EtOAc-hexanes gradient elution, TLC in 20% EtOAc-Hexanes) to give the desired title product (2.68 g, 35.2% yield) as a colorless oil. MS: M+1 = 364.7 (APCI).

**Intermediate 10. 4-(3,5-Dimethyl-cyclohexyloxy)-2-morpholin-4-yl-pyrimidine-5-carboxylic acid. Intermediate 9** (2.48 g, 6.83 mmol) was stirred with dioxane (19.2 mL) and 1N LiOH (12.8 mL) in a round bottom flask at 80°C for 90 minutes. The mix was allowed to cool to room temperature and then was slowly and carefully acidified with 1N HCl to a pH of about 1. A white solid precipitated. The mix was diluted with more H₂O and then filtered. The filter cake was rinsed with H₂O, collected, and dried overnight in the vacuum oven to give the desired title product (1.43 g, 62.2% yield) as a white solid. MS: M+1 = 336.2 (APCI). Microanalysis: calculated C-60.88%, H-7.51%, N-12.53%; found C-60.87%, H-7.67%, N-12.24%.

**Example 16. 4-(3,5-Dimethyl-cyclohexyloxy)-2-morpholin-4-yl-pyrimidine-5-carboxylic acid (2H-tetrazol-5-yl)-amide. Intermediate 10** (0.750 g, 2.24 mmol) was mixed with - but did not dissolve in anhydrous dichloromethane (11 mL). About three drops of DMF were added. Oxalyl chloride (0.213 mL, 2.46 mmol) was added via syringe. The mix bubbled and slowly became homogeneous. The reaction was stirred at room temperature for ten minutes. HPLC analysis of an aliquot quenched into MeOH/K₂CO₃ indicated complete consumption of the starting acid and the formation of a new, less-polar product. 5-Amino tetrazole (0.400 g, 4.70 mmol), triethylamine (0.654 mL, 4.70 mmol), and finally acetonitrile (11mL) were added. The reaction was stirred at 80°C for 60 minutes. HPLC analysis of an aliquot quenched into MeOH/K₂CO₃ indicated a new product. The reaction vessel was opened and allowed to boil nearly dry. The oil bath was removed and the mix was allowed to cool to room temperature. The remaining tar was taken up in a minimum of acetonitrile (~15mL). The mix was diluted with H₂O (~100mL) and acidified with 1N HCl until a fluffy white solid precipitated. The solid was filtered and rinsed with H₂O. The cake was collected and slurried in a minimum of MeOH for 30 minutes. The slurry was filtered and the cake was rinsed with a minimum of MeOH. The solid was dried overnight in a vacuum oven to give the desired title product (0.699 g, 77.6% yield) as a white solid.

| 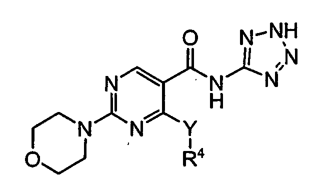 | | | | |
|---|---|---|---|---|
| Ex. | **-Y-R⁴** | **MS^{a}** | **NMR^{b} and/or combustion analysis** | **IC₅₀ (µM)** |
| 17 | 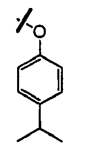 | 411.3 | 1.20 (d, *J*=6.83Hz, 6H), 2.91 (m, 1H), 3.55 (m, 4H), 3.81 (m, 2H), 7.19 (m, 2H), 7.30 (m, 2H), 8.72 (m, 1H), 11.70 (s, 1H), 16.02 (s, 1H). | 0.805 |
| | | | calculated C-54.68%, H-5.36%, N-28.53%. found C-54.56%, H-5.07%, N-28.44%. | |
| 18 | 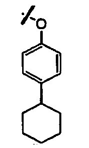 | 451.3 | 1.19-1.79 (m, 11H), 3.54 (m, 8H), 7.17 (d, J=8.54Hz, 2H), 7.27 (d, J=8.54Hz, 2H), 8.72 (s, 1H), 11.67 (s, 1H), 16.03 (s, 1H). | 0.1545 |

| | | | | |
|---|---|---|---|---|
| ^{a} MS: M+1 APCI unless otherwise indicated. ^{b}DMSO, 400 MHz, δ values reported. | | | | |

**Intermediate 11. 4-(4-Isopropyl-phenoxy)-2-methylsulfanyl-pyrimidine-5-carboxylic acid ethyl ester.** To a stirring solution of 4-isopropylphenol (5.86 g, 43.1 mmol) in anhydrous THF (100 mL) was added sodium hydride (1.03 g, 43.1 mmol) slowly and carefully. The reaction was stirred at room temperature for 20 minutes. The phenoxide solution was then poured into a stirring solution of ethyl 4-chloro-2-thiomethyl-5-pyrimidine carboxylate (10.0 g, 43.1 mmol) in anhydrous THF (100 mL). The reaction was stirred at room temperature for 15 minutes. The reaction was diluted with H₂O and was extracted several times with EtOAc. The organic extracts were combined, washed with brine, dried over Na₂SO₄, decanted, and concentrated. The concentrate was purified by silica gel chromatography (0-20% EtOAc-hexanes gradient elution, TLC in 10% EtOAc-Hexanes) to give the desired title product (9.88 g, 69.0% yield) as a light yellow oil. MS: M+1 = 333.5 (APCI).

**Intermediate 12. 4-(4-Isopropyl-phenoxy)-2-methanesulfinyl-pyrimidine-5-carboxylic acid ethyl ester.** To a stirring solution of Intermediate 11 (9.03 g, 27.2 mmol) in chloroform (91 mL) was added *m*CPBA (7.32 g, 32.6 mmol) portionwise. The exothermic reaction was moderated with an ice-water bath to keep the reaction temperature near room temperature. The reaction was then stirred at room temperature for 25 minutes. The mix was quenched with saturated aqueous NaHCO₃ and diluted with water. The dilution was extracted several times with EtOAc. The organic extracts were washed with brine, dried over Na₂SO₄, filtered through celite, and concentrated to give the desired title product as a colorless oil in quantitative yield. MS: M+1 = 349.1 (APCI).

**Intermediate 13. 4-(4-Isopropyl-phenoxy)-2-morpholin-4-yl-pyrimidine-5-carboxylic acid ethyl ester. Intermediate 12** (9.82 g, 28.2 mmol) was stirred in morpholine (12.3 mL) at 100 °C for one hour. The reaction was allowed to cool to room temperature. The reaction mix was purified by silica gel chromatography (0-30% EtOAc-hexanes gradient elution, TLC in 20% EtOAc-Hexanes) to give the desired title product (2.56 g, 24.4% yield) as a light yellow oil. MS: M+1 = 372.5 (APCI).

**Intermediate 14. 4-(4-Isopropyl-phenoxy)-2-morpholin-4-yl-pyrimidine-5-carboxylic acid. Intermediate 13** (2.36 g, 6.36 mmol) was stirred with dioxane (19.2 mL) and 1N LiOH (12.8 mL) in a round bottom flask at 80°C for ninety minutes. The mix was allowed to cool to room temperature and then was slowly and carefully acidified with 1N HCl to a pH of about 2-3. A white solid precipitated. The mix was diluted with more H₂O and then filtered. The filter cake was rinsed with H₂O, collected, and dried overnight in the vacuum oven to give the desired title product (1.81 g, 82.9% yield) as a white solid. MS: M+1 = 344.2 (APCI). Microanalysis: calculated C-62.96%, H-6.16%, N-12.24%; found C-62.95%, H-6.20%, N-12.18%.

**Example 17. 4-(4-Isopropyl-phenoxy)-2-morpholin-4-yl-pyrimidine-5-carboxylic acid (2H-tetrazol-5-yl)-amide. Intermediate 14** (0.800 g, 2.33 mmol) was mixed with - but did not dissolve in anhydrous dichloromethane (11mL). About five drops of DMF were added. Oxalyl chloride (0.222 mL, 2.56 mmol) was added via syringe. The mix bubbled and slowly became homogeneous. The reaction was stirred at room temperature for ten minutes. HPLC analysis of an aliquot quenched into MeOH/K₂CO₃ indicated complete consumption of the starting acid and the formation of a new, less-polar product. 5-Amino tetrazole (0.416 g, 4.89 mmol), triethylamine (0.680 mL, 4.89 mmol), and finally acetonitrile (11mL) were added. The reaction was stirred at 80°C for 60 minutes. HPLC analysis of an aliquot quenched into MeOH/K₂CO₃ indicated a new product. The reaction vessel was opened and allowed to boil nearly dry. The oil bath was removed and the mix was allowed to cool to room temperature. The remaining tar was taken up in a minimum of acetonitrile (~1mL). The mix was diluted with H₂O (~15mL) and acidified with 1N HCl until a fluffy white solid precipitated. The solid was filtered and rinsed with H₂O. The cake was collected and slurried in a minimum of MeOH for 30 minutes. The slurry was filtered and the cake was rinsed with a minimum of MeOH. The solid was dried overnight in a vacuum oven to give the desired title product (0.739 g, 77.4% yield) as a white solid.

**Intermediate 15. 4-Chloro-2-morpholin-4-yl-pyrimidine-5-carboxylic acid ethyl ester**. To a room temperature solution of **Intermediate 4** (3.46 g, 13.7 mmol) and DMF (~5 drops) in anhydrous dichloromethane (70 mL) was added oxalyl chloride (1.31 mL, 15.0 mmol) dropwise via syringe. The reaction effervesced. The reaction was stirred at room temperature for twenty minutes. HPLC analysis of an aliquot indicated complete consumption of the starting pyrimidine and the formation of a new, less-polar product. The reaction was diluted with saturated aqueous NaHCO₃. The dilution was extracted three times with dichloromethane. The extracts were combined, washed with brine, dried over Na₂SO₄, filtered through celite, and concentrated to give the desired title product (1.056 g, 96.5% yield), as a light yellow solid. MS: M+1 = 272.1 (APCI). ¹H-NMR (400 MHz, CDCl3) δ ppm 1.37 (t, *J*=7.08 Hz, 3H), 3.75 (m, 4H), 3.90 (m, 4H), 4.34 (q, *J*=7.08 Hz, 2H), 8.81 (s, 1H).

**Intermediate 16. 4-(4-Cyclohexyl-phenoxy)-2-morpholin-4-yl-pyrimidine-5-carboxylic acid ethyl ester.** To a stirring solution of 4-cyclohexylphenol (0.290 g, 1.62 mmol) in anhydrous THF (5 mL) was added sodium hydride (0.040 g, 1.62 mmol) slowly and carefully. The reaction was stirred at room temperature for 20 minutes. The phenoxide solution was then poured into a stirring solution of **Intermediate 15** (0.440 g, 1.62 mmol) in anhydrous THF (5 mL). The reaction was stirred at room temperature for 15 minutes. The reaction was diluted with H₂O and was extracted several times with EtOAc. The organic extracts were combined, washed with brine, dried over Na₂SO₄, filtered through celite, and concentrated. The concentrate was purified by silica gel chromatography (0-30% EtOAc-hexanes gradient elution) to give the desired title product (0.445 g, 66.9% yield) as a light yellow oil. MS: M+1 = 412.3 (APCI).

**Intermediate 17.4-(4-Cyclohexyl-phenoxy)-2-morpholin-4-yl-pyrimidine-5-carboxylic acid. Intermediate 16** (0.445 g, 1.08 mmol) was stirred with dioxane (3.0 mL) and 1N LiOH (2.25 mL) in a round bottom flask at 80°C for fifteen minutes. The mix was allowed to cool to room temperature and then was slowly and carefully acidified with 1N HCl to a pH of about 2-3. A white solid precipitated. The mix was diluted with more H₂O and then filtered. The filter cake was rinsed with H₂O, collected, and dried overnight in a vacuum oven to give the desired title product (0.369 g, 88.9% yield) as a white solid. MS: M+1 = 384.2 (APCI).

**Example 18. 4-(4-Cyclohexyl-phenoxy)-2-morpholin-4-yl-pyrimidine-5-carboxylic acid (2H-tetrazol-5-yl)-amide. Intermediate 17** (0.369 g, 0.960 mmol) was mixed with - but did not dissolve in anhydrous dichloromethane (5 mL). About three drops of DMF were added. Oxalyl chloride (0.093 mL, 1.06 mmol) was added via syringe. The mix bubbled and slowly became homogeneous. The reaction was stirred at room temperature for ten minutes. HPLC analysis of an aliquot quenched into MeOH/K₂CO₃ indicated complete consumption of the starting acid and the formation of a new, less-polar product. 5-Amino tetrazole (0.204 g, 2.40 mmol), triethylamine (0.330 mL, 2.40 mmol), and finally acetonitrile (5mL) were added. The reaction was stirred at 80°C for 60 minutes. HPLC analysis of an aliquot quenched into MeOH/K₂CO₃ indicated a new product. The reaction vessel was opened and allowed to boil nearly dry. The oil bath was removed and the mix was allowed to cool to room temperature. The remaining tar was taken up in a minimum of acetonitrile (~1mL). The mix was diluted with H₂O (~15mL) and acidified with 1N HCl until a fluffy white solid precipitated. The solid was filtered and rinsed with H₂O. The cake was collected and slurried in a minimum of MeOH for 30 minutes. The slurry was filtered and the cake was rinsed with a minimum of MeOH. The solid was dried overnight in a vacuum oven to give the desired title product (0.246 g, 56.9% yield) as a white solid.

| 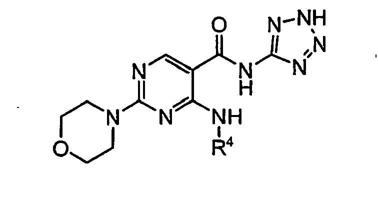 | | | | |
|---|---|---|---|---|
| **Ex.** | **-R⁴** | **MS^{a}** | NMR^{b} and/or combustion analysis | **IC₅₀ (µM)** |
| 19 | 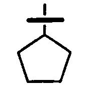 | 360.3 | 1.37-1.45 (m, 2H), 1.53-1.70 (m, 4H), 1.96-2.04 (m, 2H), 3.61-3.63 (m, 4H), 3.75-3.78 (m, 4H), 7.28-4.34 (m, 1H), 8.55 (d, *J*=6.83Hz), 8.76 (s, 1H) | 32.72 |
| 20 | 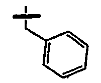 | 382.2 | 3.57-3.60 (m, 4H), 3.73-3.76 (m, 4H), 4.62-4.64 (m, 2H), 7.22-7.30 (m, 1H), 7.30-7.35 (m, 4H), 8.78 (s, 1H), 8.87 (bt, 1H,*J*=5.61Hz) | 39.09 |
| 21 | 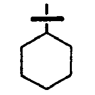 | 374.2 | 1.21-1.40 (m, 5H), 1.53-1.56 (m, 1H), 1.65-1.68 (m, 2H), 1.88-1.92 (m, 2H), 3.61-3.63 (m, 4H) 3.68-3.75 (m, 4H), 3.93-3.98 (m, 1H), 8.53 (d, 1H, J=7.3 Hz), 8.76 (s, 1H) | 28.59 |

**Intermediate 18: 4-Cyclopentylamino-2-methylsulfanyl-pyrimidine-5-carboxylic acid ethyl ester.** To a stirred solution of ethyl 4-chloro-2-methylthio-5-pyrimidine carboxylate (2.00g, 8.59 mmol) in methylene chloride was added triethylamine (3.60 mL, 25.8 mmol) followed by cyclopentylamine (1.0 mL, 10.12 mmol). The reaction solution was heated to reflux for 45 min. HPLC analysis of an aliquot indicated complete consumption of the starting pyrimidine and the formation of a new product. Reaction was poured into 1M HCl and diluted with methylene chloride. Organic layer was washed with water, brine, dried over MgSO₄, filtered and concentrated to a beige solid. (2.3614 g, 97%).

**Intermediate 19: 4-Cyclopentylamino-2-methanesulfinyl-pyrimidine-5-carboxylic acid ethyl ester. Intermediate 19** was prepared in a manner similar **to that of Intermediate 8 using Intermediate 18** (2.36 g, 8.39 mmol) and *m*CPBA (2.14g, 1.14 mmol) to provide a pale yellow oil (2.456 g, 98%).

**Intermediate 20: 4-Cyclopentylamino-2-morpholin-4-yl-pyrimidine-5-carboxylic acid ethyl ester. Intermediate 20** was prepared in a manner similar to that of **Intermediate 9** using **Intermediate 19** (2.45 g, 8.24 mmol) and morpholine (20 mL, 229.3 mmol) to provide a crude off-white solid. This was purified by silica gel chromatography (25% ether/hexanes) to provide a fluffy white solid (2.52 g, 95%).

**Intermediate 21: 4-Cyclopentylamino-2-morpholin-4-yl-pyrimidine-5-carboxylic acid. Intermediate 21** was prepared in a manner similar to that of **Intermediate 10** using **Intermediate 20** (0.971 g, 3.03 mmol) and 6.2 mL of 1M LiOH to provide a white solid (0.700 g, 79%).

**Example 19. 4-Cyclopentylamino-2-morpholin-4-yl-pyrimidine-5-carboxylic acid (1H-tetrazol-5-yl)-amide. Example 19** was prepared in a manner similar to that of **Example 15** using **Intermediate 21** (0.129 g, 0.441 mmol) to provide the title product as a white solid (0.125 g, 79%). MS: M+1= 360.3 (APCI). ¹H-NMR (400MHz, d6-DMSO) δ 1.37-1.45 (m, 2H), 1.53-1.70 (m, 4H), 1.96-2.04 (m, 2H), 3.61-3.63 (m, 4H), 3.75-3.78 (m, 4H), 7.28-4.34 (m, 1H), 8.55 (d, J=6.83Hz), 8.76 (s, 1H).

The title compounds of Examples 20 and 21 were synthesized in a manner analogous to Example 19 by replacing cyclopentyl amine with benzyl amine or cyclohexylamine, respectively.

### Example 20. 4-Benzylamino-2-morpholin-4-yl-pyrimidine-5-carboxylic acid (1H-tetrazol-5-yl)-amide.

### Example 21. 4-Cyclohexylamino-2-morpholin-4-yl-pyrimidine-5-carboxylic acid (1H-tetrazol-5-yl)-amide.

| | | | | |
|---|---|---|---|---|
| 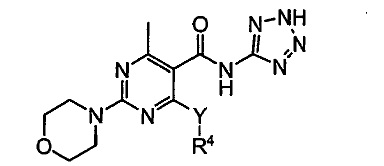 | | | | |

| **Ex.** | **-Y-R**⁴ | **MS^{a}** | NMR^{b} and/or combustion analysis | **IC₅₀ (µM)** |
|---|---|---|---|---|
| 22 | 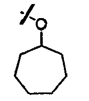 | 403.2 | 1.48 (m, 8H), 1.71 (m, 2H), 1.90 (m, 2H), 2.26 (s, 3H), 3.63 (m, 4H), 3.71 (m, 4H), 5.20 (m, 1H), 11.86 (s, 1H). | 8.435 |

| | | | | |
|---|---|---|---|---|
| ^{a} MS: M+1 APCI unless otherwise indicated. ^{b}DMSO, 400 MHz, δ values reported. | | | | |

**Intermediate 22. 1-[1-(Dimethylamino)ethylidene]-2-methylisothiourea hydroiodide**. A 5 L 3-neck round bottom flask fixed with an overhead stirrer, reflux condenser, nitrogen inlet, and heating mantle was charged with thiourea (162.8 g, 2.14 mol) and anhydrous dichloromethane (2.4 L). N,N-dimethyl acetamide dimethyl acetal (399.9 g, 3 mol, 1.4 eq.) was added to the flask. The reaction mixture was heated to reflux under nitrogen for two hours and then removed from heat and concentrated to dryness to give an orange-colored solid. The orange solid was suspended in anhydrous THF (1.5 L) and transferred to a 5 L 3-neck round bottom flask fixed with an overhead stirrer, temperature probe, and an addition funnel with an N₂ inlet. To this stirred suspension was added MeI (1668 mL) slowly in portions. The first portion (80 mL) resulted in an exotherm and the solution temperature increased from 23 to 26 °C. The reaction mixture was cooled externally with ice-water and another portion of MeI (20 mL) was added. As addition of MeI continued, the temperature dropped and another portion of MeI (780 mL) was added without an observed exotherm. The ice bath was removed and the final portion of MeI (788 mL) was added without an observed exotherm. After the addition was completed, the reaction mixture was stirred under N₂ overnight and then transferred to the freezer (-20 °C) for 72 hours. The reaction mixture was allowed to warm to room temperature, filtered, and the solid was washed with Et₂O (2 x 500 mL) providing the product as a tan solid. The solids were dried under vacuum at room temperature overnight to give the desired product (556.6 g, 91.0 %) as a tan solid.

**Intermediate 23. Ethyl-4-hydroxy-6-methyl-2-(methylthio)-5-pyrimidinecarboxylate.** A 12 L 3-neck round bottom flask fixed with an overhead stirrer, an addition funnel with an N₂ inlet, and thermometer, was charged with **Intermediate 22** (556.6 g, 1.94 mmol) and anhydrous dichloromethane (5 L). The reaction mixture was cooled to ~2 °C (ice-bath) while stirring under N₂, and ethyl malonyl chloride (350 g, 2.33 mol) was added. After the addition, the reaction was stirred at ~2 °C for an additional 1.5 hours. To this suspension was added triethyl amine (648 mL, 4.66 mol) dropwise maintaining the reaction below 5 °C during the addition (2.5 h). Upon completion of the addition, the reaction was allowed to warm to room temperature as was stirred under N₂ overnight. The reaction mixture was acidified to pH~4 by adding 525 mL of 10% aqueous HCl solution. The organic layer was separated and washed with brine (1 x 1050 mL). Another 750 mL of brine was added to the organic layer and the pH of the brine layer was adjusted from 5-5 to about 7 by adding saturated aqueous NaHCO₃ solution (~225 mL) in portions. The organic layer was separated, dried over MgSO₄ for 1.5 hours, filtered, and concentrated by rotovap to provide a gummy orange solid which was dried under high vacuum at room temperature overnight. The gummy wet residue (~579.5 g) was triturated with 1.2 L of hexanes:EtOAc (1:1) for 3 hours. The mixture was filtered providing a gummy yellow solid which was dried under high vacuum overnight. The yellow solid (~400 g) was taken up in 2.8 L of dichloromethane and the resulting heterogenous suspension was filtered. The clear dichloromethane solution was chromatographed over SiO₂ with 0-10% EtOAc-dichloromethane. The product rich fractions were collected, concentrated, and re-chromatographed with 0-10% EtOAc-dichloromethane. The product rich fractions were concentrated, triturated with 800 mL hexanes:EtOAc (1:1) overnight, and filtered. The yellow solid was dried under high vacuum overnight to give the desired product (113.6 g, 26%)as a pale yellow solid.

**Intermediate 24. 4-Cycloheptyloxy-6-methyl-2-methylsulfanyl-pyrimidine-5-carboxylic acid ethyl ester. Intermediate 23** (3.00 g, 13.2 mmol) was combined with cycloheptanol (1.75 mL, 14.5 mmol), DEAD (3.12 mL, 19.8 mmol), and triphenyl phosphine (5.19 g, 19.8 mmol) in THF (60 mL). The reaction was stirred at room temperature for 18 hours. HPLC analysis indicated a new product along with excess triphenyl phosphine and triphenylphosphine oxide. The reaction mixture was concentrated *en vacuo.* The concentrate was purified by silica gel chromatography (0-17% EtOAc-hexanes gradient elution, TLC in 10% EtOAc-Hexanes) to give the desired title product (3.64 g, 85% yield) as a light pink oil. MS: M+1 = 325.1 (APCI).

**Intermediate 25. 4-Cycloheptyloxy-2-methanesulfinyl-6-methyl-pyrimidine-5-carboxylic acid ethyl ester.** To a stirring solution of **Intermediate 24** (3.252 g, 10.04 mmol) in chloroform (50 mL) was added *m*CPBA (2.71 g, 12.1 mmol) portionwise. The reaction was then stirred at room temperature for 40 minutes. Another portion of *m*CPBA (350 mg) was added and the reaction was stirred at room temperature for 15 minutes. The mix was quenched with saturated aqueous NaHCO₃ and diluted with water. The dilution was extracted several times with dichloromethane. The organic extracts were washed with brine, dried over Na₂SO₄, filtered through celite, and concentrated to give the desired title product (3.57 g) as a colorless oil. The product was carried on without further purification. MS: M+1 = 341.1 (APCI).

**Intermediate 26. 4-Cycloheptyloxy-6-methyl-2-morpholin-4-yl-pyrimidine-5-carboxylic acid ethyl ester. Intermediate 25** (0.298 g, 0.876 mmol) was stirred in morpholine (0.381 mL) at 100 °C for one hour. The reaction was allowed to cool to room temperature The reaction mix was purified by silica gel chromatography (0-50% EtOAc-hexanes gradient elution, TLC in 20% EtOAc-Hexanes) to give the desired title product (0.274 g, 86.2% yield) as colorless oil. MS: M+1 = 364.1 (APCI). Microanalysis: calculated C-62.79%, H-8.04%, N-11.56%; found C-62.42%, H-8.13%, N-11.59%.

**Intermediate 27.4-Cycloheptyloxy-6-methyl-2-morpholin-4-yl-pyrimidine-5-carboxylic acid. Intermediate 6** (0.237 g, 0.653 mmol) was stirred with dioxane (3 mL) and 1N LiOH (2 mL) in a round bottom flask at 80°C for six hours. The mix was allowed to cool to room temperature and then was slowly and carefully acidified with 1N HCl to pH~1. The acid mix was extracted several times with EtOAc.. The organic extracts were combined, washed with brine, dried over Na₂SO₄, filtered through celite, and concentrated to give the desired title product (0.218 g, 100% yield) as an oil that solidified on standing. MS: M+1 = 336.1 (APCI).

**Example 22. 4-Cycloheptyloxy-6-methyl-2-morpholin-4-yl-pyrimidine-5-carboxylic acid (2H-tetrazol-5-yl)-amide. Intermediate 27** (0.131 g, 0.391 mmol) was mixed with - but did not dissolve in anhydrous dichloromethane (2.5 mL). One drop of DMF was added. Oxalyl chloride (0.0406 mL, 0.469 mmol) was added via syringe. The mix bubbled and slowly became homogeneous. The reaction was stirred at room temperature for ten minutes. HPLC analysis of an aliquot quenched into MeOH/K₂CO₃ indicated complete consumption of the starting acid and the formation of a new, less-polar product. 5-Amino tetrazole (0.0565 g, 0.665 mmol), triethylamine (0.114 mL, 0.821 mmol), and finally acetonitrile (2.5 mL) were added. The reaction was stirred at 80°C for 30 minutes. HPLC analysis of an aliquot quenched into MeOH/K₂CO₃ indicated a new product. The oil bath was removed and the mix was allowed to cool to room temperature. The mix was acidified with 1N HCl, diluted with H2O, and extracted several times with EtOAc. The organic extracts were combined, washed with brine, dried over Na₂SO₄, filtered through celite, and concentrated. The crude product was purified by preparative-scale, reverse phase HPLC to give the desired title product (0.040 g, 24.4%) as a white solid.

**Intermediate 4** was also prepared according to the following procedure: Morpholinoformamidine hydrobromide (10.00 g, 77.42 mmol) was stirred with diethyl ethoxymethylenemalonate (23.5 mL, 116 mmol) and sodium acetate (14.0 g, 2.20 mmol) in DMF (240 mL) at 110°C for 18 hours. The reaction slowly turned from light yellow to red over the first several hours. HPLC analysis indicated formation of the desired product. The reaction mix was concentrated *en vacuo.* The remaining residue was slurried in H₂O (~150 mL) at room temperature for one hour to give a beige solid precipitate. The solid was filtered, rinsed with H₂O, and dried in the vacuum oven. The dried cake was then slurried in Et₂O (~70 mL) at room temperature for twenty minutes. The mixture was filtered and the cake was rinsed with a minimum of Et₂O. The solid was collected and dried overnight under vacuum to give the desired product, 4-hydroxy-2-morpholin-4-yl-pyrimidine-5-carboxylic acid ethyl ester (**Intermediate 4**, 7.251 g, 59.9% yield), as a fluffy beige solid.

### BIOLOGICAL EXAMPLE 1

### PI3Kγ Protein Expression and Purification Protocol

Spodtera frugiperda cells, grown in ESF921 media, were co-infected with baculovirus expressing a glu-tagged p101 and baculovirus expressing an HA-tagged p110γ, at a 3:1 ratio of p101 baculovirus to p110γ baculovirus. Sf9 cells were grown to 1 × 10⁷ total cells/mL in 10L bioreactors and harvested 48-72 hours post infection. Samples of infected cells were then tested for expression of p101/p110γ PI3 kinase by immunoprecipitation and Western Blot analysis methods (see below).

To purify PI3Kγ, 4 volumes of room temperature hypotonic lysis buffer (1 mM MgCl₂, 1 mM DTT, 5 mM EGTA, 1 mM Pefabloc, 0.5 µM aprotinin, 5 µM leupeptin, 2 µM pepstatin, 5 µM E64, pH 8) per gram of cell paste, was poured onto frozen cell pellets with stirring, then lysed in a nitrogen "bomb" at 400 psi (599HC T316, Parr Instrument Co, Moline, IL). NaCl was added to 150 mM, and sodium cholate was added to 1% and mixed for another 45 minutes. The lysates were clarified by centrifugation for 25 minutes at 14,000 rpm. The lysates were then loaded over anti-glu-linked Protein-G Sepaharose beads (Covance Research Products, Richmond, CA) using 20 mL resin/50 g cell paste. The column was washed with 15 volumes of wash buffer (1 mM DTT, 0.2 mM EGTA, 1 mM Pefabloc, 0.5 µM aprotinin, 5 µM leupeptin, 2 µM pepstatin, 5 µM E64, 150 mM NaCl, 1% sodium cholate, pH 8). PI3Kγ was eluted with 6 column volumes of wash buffer that contain 100 µg/mL of a peptide that competes for binding of the glu tag. The column fractions with the eluted protein (determined by taking OD₂₈₀ readings) were collected and dialyzed in 0.2 mM EGTA, 1 mM DTT, 1 mM Pefabloc, 5 µM leupeptin, 0.5% sodium cholate, 150 mM NaCl, and 50% glycerol, pH 8. The fractions were stored at -80°C until further use.

### BIOLOGICAL EXAMPLE 2

### G Protein Subunits Expression

Spodtera frugiperda cells were coinfected with baculovirus expressing a glu-tagged G protein β₁ and baculovirus expressing a G protein β₂, at a 1:1 ratio of glu-tagged G protein β₁ baculovirus to G protein β₂ baculovirus. Sf9 cells are grown in 10 L bioreactors and harvested 48-72 hours post infection. Samples of infected cells were tested for G protein β₁/β₂ expression by Western Blot analysis, as described below. Cell lysates were homogenized and loaded onto a column of glu-tagged beads as in Biological Example 1 and competed off the column with a glu peptide and processed as described in Biological Example 1.

### BIOLOGICAL EXAMPLE 3

### Western Blot Analysis

Protein samples were run on an 8% Tris-Glycine gel and transferred to a 45 µM nitrocellulose membrane. The blots were then blocked with 5% bovine serum albumin (BSA) and 5% ovalbumin in TBST (50 mM Tris, 200 mM NaCl, 0.1% Tween 20, ph 7.4) for 1 hour at room temperature, and incubated overnight at 4°C with primary antibody diluted 1:1000 in TBST with 0.5% BSA. The primary antibodies for the p110γ, p110α, p110β, p85α, G protein β₁, and G protein γ₂ subunits were purchased from Santa Cruz Biotechnology, Inc., Santa Cruz, CA. The p101 subunit antibodies were developed at Research Genetics, Inc., Huntsville, AL based on a p101 peptide antigen.

After incubation with the primary antibody, the blots were washed in TBST and incubated for 2 hours at room temperaure with goat-anti-rabbit HRP conjugate (Bio-Rad Laboratories, Inc., Hercules, CA, product Number 170-6515), diluted 1:10,000 in TBST with 0.5% BSA. The antibodies were detected with ECL^{™} detection reagents (Amersham Biosciences Corp., Piscataway, New Jersey) and quantified on a Kodak ISO400F scanner.

### BIOLOGICAL EXAMPLE 4

### Immunoprecipitation

100 µL of cell paste from Biological Example 1 or 2 was thawed and lysed on ice with 400 µL of hypotonic lysis buffer (25 mM tris, 1 mM DTT, 1 mM EDTA, 1 mM Pefabloc, 5 µM leupeptin, 5 µM E-64 (Roche), 1% Nonidet P40, pH 7.5-8). The lysate was incubated for 2 hours at room temperature with glu-tagged beads (Covance Research Products, Cambridge, England, product Number AFC-115P). The beads were washed 3 times in wash buffer (20 mM Tris, pH 7.8-8, 150 mM NaCl₂, 0.5% NP40) and the protein eluted off the beads by heating in 2 times sample buffer (Invitrogen Corporation, Carlsbad, CA, product Number LC 1676).

### BIOLOGICAL EXAMPLE 5

### PI3Kγ In Vitro Kinase Assay

The inhibitory properties of the compounds in Table 1 were assayed in an in vitro PI3K assay. In a 96-well polypropylene plate, each well was spotted with 2 µL of 50 times the desired final concentration of compound in DMSO. Purified recombinant p101/p110γ protein (0.03 µg; -2.7 nM) and G protein β₁/γ₂ subunits (0.09 µg; -57.7 nM) for each reaction was combined in the assay buffer (30 mM HEPES, 100 mM NaCl, 1 mM EGTA, and 1 mM DTT). ATP and [γ-³²P-ATP] (0.09 µCi) were added to this mixture so that the final ATP concentration in the reaction was 20 µM. Lipid micelles were formed by sonicating phosphatidylinositol-4,5-diphosphate (PIP₂), phosphatidylethanolamine (PE), and Na-cholate in the assay buffer for 10 minutes, adding MgCl₂ and incubating on ice for 20 minutes, for final concentrations of 25 µM PIP₂, 300 µM PE, 0.02% Na-cholate, and 10 mM MgCl₂ in the reaction. The reactions were started by adding equal volumes lipid and enzyme mixture in a total volume of 50 µL, allowed to run for 20 minutes at room temperature, and stopped with 100 µL 75 mM H₃PO₄. The lipid product was transferred to a glass fiber filter plate and washed with 75 mM H₃PO₄ several times. The presence of radioactive lipid product (PIP₃) was measured by adding Wallac Optiphase mix to each well and counting in a Wallac 1450 Trilux plate reader (PerkinElmer Life Sciences Inc., Boston, MA 02118). The IC₅₀ for each compound tested is reported in µM in the Tables above.

### BIOLOGICAL EXAMPLE 6

### Murine Collagen Induced Arthritis

Bovine type II collagen (University of Utah) was diluted with 0.01N acetic acid to a concentration of 2 mg/mL and emulsified with an equal volume of Freund's complete adjuvant (Difco, Detroit, Michigan) supplemented with 1 mg/mL Mycobacterium tuberculosis Hra37. Age-matched (8-12 weeks) female DBA/1 LacJ mice (Jackson Laboratories, Bar Harbor, Maine) were immunized with 100 µL of emulsion (100 µg collagen) intradermally at the base of the tail. On Day 28 after immunization, the mice were given 50 µg Lipopolysaccharide (Sigma Aldrich, St Louis, Missouri) intraperitoneally (IP) in 100 µL saline. The compound of Example 1 (4-Cycloheptyloxy-2-morpholin-4-yl-pyrimidine-5-carboxylic acid (2H-tetrazol-5-yl)-amide) was dosed at 30 mg/kg q.d. by oral gavage for two weeks beginning on day 27. The development of arthritis was assessed on Days 27, 31, 34, 37, and 41. A clinical score was given to each limb using the following scale: (0) normal, (1) erythema and edema, (2) joint distortion, (3) joint ankylosis. Edema measurements on front paws, rear paws, and ankles were taken using constant tension calipers (Dyer, Lancaster, Pennsylvania). The compound of Example 1 at 30 mg/kg q.d. inhibited paw edema in this model by 52%, and clinical score by 35%.

### BIOLOGICAL EXAMPLE 7

### SCW- Streptococcal Cell Wall-Induced Paw Edema

Female Lewis rats (~150 g) received 6 µg SCW in 10 µl Dulbecco's PBS by an intraarticular injection to the right tibiotalar joint on day 0. On day 21, the recurrence response was initiated with 100 µg SCW (250 µl, i.v.). Vehicle (0.5% hydroxypropylmethylcellulose/0.2% Tween 80) or the compound of Example 1 (4-Cycloheptyloxy-2-morpholin-4-yl-pyrimidine-5-carboxylic acid (2H-tetrazol-5-yl)-amide) was given orally (10 ml/kg volume), q.d., 1 hr prior to SCW, i.v., and on subsequent days. Baseline volumes of the sensitized hind paw were measured by a mercury displacement plethysmometer before disease initiation on day 21, and subtracted from subsequent assessments (made 24 hrs after each dose) to determine the Δ value (the difference between paw volume prior to disease initiation on day 21 and the paw volume 24 hours after each dose). The compound of Example 1 was dosed at 10, 30 and 100 mg/kg q.d. by oral gavage for four days beginning on day 21. The compound of Example 1 inhibited paw edema in this model with an ID₅₀ of 16.3 mg/kg.

It is understood that the examples and embodiments described herein are for illustrative purposes only.

## Claims

1. A compound of Formula I: 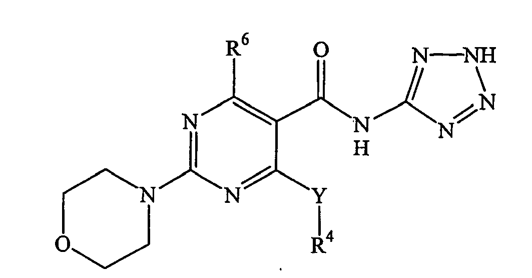 or a pharmaceutically acceptable salt thereof;
wherein:
R⁶ is hydrogen or methyl;
Y is O, NH, or S;
R⁴ is selected from the group consisting of: a C₂-C₆ alkyl, a -L-C₃-C₈ cycloalkyl, an adamantyl, a 5 or 6-membered heterocycloalkyl, and -J-R^{c};
wherein R^{c} is a phenyl group or a naphthyl group,
wherein L is absent or a C₁-C₃alkylene, and
wherein J is absent or is a C₁-C₄-alkylene.

2. The compound of claim 1, wherein Y is O, R⁶ is hydrogen, and R⁴ is a C₃-C₈ cycloalkyl.

3. The compound of claim 2, wherein said compound is:
4-Cycloheptyloxy-2-morpholin-4-yl-pyrimidine-5-carboxylic acid (2H-tetrazol-5-yl)-amide;
4-Cyclopentyloxy-2-morpholin-4-yl-pyrimidine-5-carboxylic acid (2H-tetrazol-5-yl)-amide;
4-Cyclohexyloxy-2-morpholin-4-yl-pyrimidine-5-carboxylic acid (2H-tetrazol-5-yl)-amide;
4-Cyclooctyloxy-2-morpholin-4-yl-pyrimidine-5-carboxylic acid (2H-tetrazol-5-yl)-amide;
4-(2-Cyclohexyl-ethoxy)-2-morpholin-4-yl-pyrimidine-5-carboxylic acid (2H-tetrazol-5-yl)-amide;
4-Cyclohexylmethoxy-2-morpholin-4-yl-pyrimidine-5-carboxylic acid (2H-tetrazol-5-yl)-amide;
4-(3,3-Dimethyl-cyclohexyloxy)-2-morpholin-4-yl-pyrimidine-5-carboxylic acid (2H-tetrazol-5-yl)-amide; and
4-(3,5-Dimethyl-cyclohexyloxy)-2-morpholin-4-yl-pyrimidine-5-carboxylic acid (2H-tetrazol-5-yl)-amide.

4. The compound of claim 1, wherein said compound is selected from the group consisting of:
4-(Adamantan-2-yloxy)-2-morpholin-4-yl-pyrimidine-5-carboxylic acid (2H-tetrazol-5-yl)-amide; and
4-(Bicyclo[2.2.1]hept-2-yloxy)-2-morpholin-4-yl-pyrimidine-5-carboxylic acid (2H-tetrazol-5-yl)-amide.

5. The compound of claim 1, wherein Y is O, R⁶ is hydrogen, and R⁴ is a C₃-C₈ cycloalkyl or a C₁-C₃alkylene-C₃-C₈ cycloalkyl.

6. The compound of claim 1, wherein Y is O, R⁶ is hydrogen, and R⁴ is a 5 or 6-membered heterocycloalkyl.

7. The compound of claim 1, wherein R⁴ is a tetrahydropyranyl.

8. The compound of claim 1, wherein Y is O, R⁶ is hydrogen, and R⁴ is a phenyl group.

9. The compound of claim 8, wherein said compound is selected from the group consisting of:
4-(4-Isopropyl-phenoxy)-2-morpholin-4-yl-pyrimidine-5-carboxylic acid (2H-tetrazol-5-yl)-amide; and
4-(4-Cyclohexyl-phenoxy)-2-morpholin-4-yl-pyrimidine-5-carboxylic acid (2H-tetrazol-5-yl)-amide.

10. The compound of claim 1, wherein Y is NH, and R⁶ is hydrogen.

11. Use of a compound according to claim 1 in the manufacture of a pharmaceutical composition comprising a therapeutically effective amount of a compound of claim 1 and a pharmaceutically acceptable carrier for the treatment of a disease selected from the group consisting of: rheumatoid arthritis, ankylosing spondylitis, osteoarthritis, psoriatic arthritis, psoriasis, inflammatory diseases, and autoimmune diseases.

12. The use of claim 11, wherein said compound is a compound of any one of claims 1-10.

13. A pharmaceutical composition comprising:
a therapeutically effective amount of a compound of claim 1 and a pharmaceutically acceptable carrier.

14. A pharmaceutical composition comprising:
a therapeutically effective amount of a compound of any one of claim 1-10
and a pharmaceutically acceptable carrier.

15. A pharmaceutical composition comprising a therapeutically effective amount of a compound selected from the group consisting of:
4-Cycloheptyloxy-2-morpholin-4-yl-pyrimidine-5-carboxylic acid (2H-tetrazol-5-yl)-amide;
4-Cyclopentyloxy-2-morpholin-4-yl-pyrimidine-5-carboxylic acid (2H-tetrazol-5-yl)-amide;
4-Cyclohexyloxy-2-morpholin-4-yl-pyrimidine-5-carboxylic acid (2H-tetrazol-5-yl)-amide;
4-Cyclooctyloxy-2-morpholin-4-yl-pyrimidine-5-carboxylic acid (2H-tetrazol-5-yl)-amide;
4-(2-Cyclohexyl-ethoxy)-2-morpholin-4-yl-pyrimidine-5-carboxylic acid (2H-tetrazol-5-yl)-amide;
4-Cyclohexylmethoxy-2-morpholin-4-yl-pyrimidine-5-carboxylic acid (2H-tetrazol-5-yl)-amide;
4-(3,3-Dimethyl-cyclohexyloxy)-2-morpholin-4-yl-pyrimidine-5-carboxylic acid (2H-tetrazol-5-yl)-amide;
4-(3,5-Dimethyl-cyclohexyloxy)-2-morpholin-4-yl-pyrimidine-5-carboxylic acid (2H-tetrazol-5-yl)-amide;
4-(Adamantan-2-yloxy)-2-morpholin-4-yl-pyrimidine-5-carboxylic acid (2H-tetrazol-5-yl)-amide;
4-(Bicyclo[2.2.1]hept-2-yloxy)-2-morpholin-4-yl-pyrimidine-5-carboxylic acid (2H-tetrazol-5-yl)-amide;
4-(4-Isopropyl-phenoxy)-2-morpholin-4-yl-pyrimidine-5-carboxylic acid (2H-tetrazol-5-yl)-amide; and
4-(4-Cyclohexyl-phenoxy)-2-morpholin-4-yl-pyrimidine-5-carboxylic acid (2H-tetrazol-5-yl)-amide.

## Patentansprüche

1. Verbindung der Formel I: 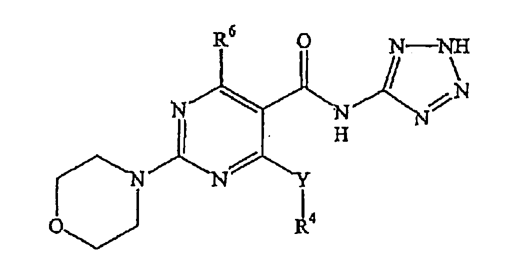 oder ein pharmazeutisch annehmbares Salz davon;
worin:
R⁶ Wasserstoff oder Methyl ist;
Y O, NH oder S ist;
R⁴ ausgewählt ist aus der Gruppe, bestehend aus: einem C₂-C₆-Alkyl, einem -L-C₃-C₈-Cycloalkyl, einem Adamantyl, einem 5- oder 6-gliedrigen Heterocycloalkyl und -J-R^{c};
worin R^{c} eine Phenylgruppe oder eine Naphthylgruppe ist,
worin L nicht vorhanden oder ein C₁-C₃-Alkylen ist, und
worin J nicht vorhanden oder ein C₁-C₄-Alkylen ist.

2. Verbindung nach Anspruch 1, worin Y O ist, R⁶ Wasserstoff ist und R⁴ ein C₃-C₈-Cycloalkyl ist.

3. Verbindung nach Anspruch 2, wobei die Verbindung ist:
4-Cycloheptyloxy-2-morpholin-4-yl-pyrimidin-5-carbonsäure(2H-tetrazol-5-yl)amid;
4-Cyclopentyloxy-2-morpholin-4-yl-pyrimidin-5-carbonsäure(2H-tetrazol-5-yl)amid;
4-Cyclohexyloxy-2-morpholin-4-yl-pyrimidin-5-carbonsäure(2H-tetrazol-5-yl)amid;
4-Cyclooctyloxy-2-morpholin-4-yl-pyrimidin-5-carbonsäure(2H-tetrazol-5-yl)amid;
4-(2-Cyclohexylethoxy)-2-morpholin-4-yl-pyrimidin-5-carbonsäure(2H-tetrazol-5-yl)amid;
4-Cyclohexylmethoxy-2-morpholin-4-yl-pyrimidin-5-carbonsäure(2H-tetrazol-5-yl)amid;
4-(3,3-Dimethylcyclohexyloxy)-2-morpholin-4-yl-pyrimidin-5-carbonsäure(2H-tetrazol-5-yl)amid; und
4-(3,5-Dimethylcyclohexyloxy)-2-morpholin-4-yl-pyrimidin-5-carbonsäure(2H-tetrazol-5-yl)amid.

4. Verbindung nach Anspruch 1, wobei die Verbindung ausgewählt ist aus der Gruppe, bestehend aus:
4-(Adamantan-2-yloxy)-2-morpholin-4-yl-pyrimidin-5-carbonsäure(2H-tetrazol-5-yl)amid; und
4-(Bicyclo[2.2.1]hept-2-yloxy)-2-morpholin-4-yl-pyrimidin-5-carbonsäure(2H-tetrazol-5-yl)amid.

5. Verbindung nach Anspruch 1, worin Y O ist, R⁶ Wasserstoff ist und R⁴ ein C₃-C₈-Cycloalkyl oder ein C₁-C₃-Alkylen-C₃-C₈-cycloalkyl ist.

6. Verbindung nach Anspruch 1, worin Y O ist, R⁶ Wasserstoff ist und R⁴ ein 5- oder 6-gliedriges Heterocycloalkyl ist.

7. Verbindung nach Anspruch 1, worin R⁴ Tetrahydropyranyl ist.

8. Verbindung nach Anspruch 1, worin Y O ist, R⁶ Wasserstoff ist und R⁴ eine Phenylgruppe ist.

9. Verbindung nach Anspruch 8, wobei die Verbindung ausgewählt ist aus der Gruppe, bestehend aus:
4-(4-Isopropylphenoxy)-2-morpholin-4-yl-pyrimidin-5-carbonsäure(2H-tetrazol-5-yl)amid; und
4-(4-Cyclohexylphenoxy)-2-morpholin-4-yl-pyrimidin-5-carbonsäure(2H-tetrazol-5-yl)amid.

10. Verbindung nach Anspruch 1, worin Y NH ist und R⁶ Wasserstoff ist.

11. Verwendung einer Verbindung gemäß Anspruch 1 in der Herstellung einer pharmazeutischen Zusammensetzung, umfassend eine therapeutisch wirksame Menge einer Verbindung nach Anspruch 1 und einen pharmazeutisch annehmbaren Träger, zur Behandlung einer Krankheit, ausgewählt aus der Gruppe, bestehend aus: rheumatoider Arthritis, Spondylarthritis, Osteoarthritis, Arthritis psoriatica, Psoriasis, Entzündungserkrankungen und Autoimmunerkrankungen.

12. Verwendung nach Anspruch 11, wobei die Verbindung eine Verbindung nach einem der Ansprüche 1-10 ist.

13. Pharmazeutische Zusammensetzung, umfassend:
eine therapeutisch wirksame Menge einer Verbindung nach Anspruch 1 und einen pharmazeutisch annehmbaren Träger.

14. Pharmazeutische Zusammensetzung, umfassend:
eine therapeutisch wirksame Menge einer Verbindung nach einem der Ansprüche 1-10 und einen pharmazeutisch annehmbaren Träger.

15. Pharmazeutische Zusammensetzung, umfassend eine therapeutisch wirksame Menge einer Verbindung, ausgewählt aus der Gruppe, bestehend aus:
4-Cycloheptyloxy-2-morpholin-4-yl-pyrimidin-5-carbonsäure(2H-tetrazol-5-yl)amid;
4-Cyclopentyloxy-2-morpholin-4-yl-pyrimidin-5-carbonsäure(2H-tetrazol-5-yl)amid;
4-Cyclohexyloxy-2-morpholin-4-yl-pyrimidin-5-carbonsäure(2H-tetrazol-5-yl)amid;
4-Cyclooctyloxy-2-morpholin-4-yl-pyrimidin-5-carbonsäure(2H-tetrazol-5-yl)amid;
4-(2-Cyclohexylethoxy)-2-morpholin-4-yl-pyrimidin-5-carbonsäure(2H-tetrazol-5-yl)amid;
4-Cyclohexylmethoxy-2-morpholin-4-yl-pyrimidin-5-carbonsäure(2H-tetrazol-5-yl)amid;
4-(3,3-Dimethylcyclohexyloxy)-2-morpholin-4-yl-pyrimidin-5-carbonsäure(2H-tetrazol-5-yl)amid;
4-(3,5-Dimethylcyclohexyloxy)-2-morpholin-4-yl-pyrimidin-5-carbonsäure(2H-tetrazol-5-yl)amid;
4-(Adamantan-2-yloxy)-2-morpholin-4-yl-pyrimidin-5-carbonsäure(2H-tetrazol-5-yl)amid;
4-(Bicyclo[2.2.1]hept-2-yloxy)-2-morpholin-4-yl-pyrimidin-5-carbonsäure(2H-tetrazol-5-yl)amid;
4-(4-Isopropylphenoxy)-2-morpholin-4-yl-pyrimidin-5-carbonsäure (2H-tetrazol-5-yl) amid; und
4-(4-Cyclohexylphenoxy)-2-morpholin-4-yl-pyrimidin-5-carbonsäure(2H-tetrazol-5-yl)amid.

## Revendications

1. Composé de Formule I : 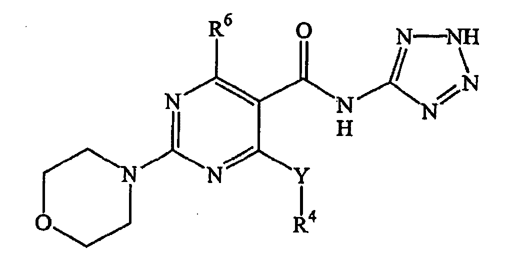 ou sel pharmaceutiquement acceptable correspondant ; formule dans laquelle :
R⁶ représente l'hydrogène ou méthyle ;
Y représente O, NH ou S ;
R⁴ est sélectionné dans le groupe consistant en :
alkyle en C₂-C₆, -L- (cycloalkyle en C₃-C₈), adamantyle, hétérocycloalkyle ayant 5 ou 6 éléments et -J-R^{c} ; où
R^{c} représente un groupe phényle ou un groupe naphtyle ;
L est absent ou représente alkylène en C₁-C₃ ; et
J est absent ou représente alkylène en C₁-C₄.

2. Composé selon la revendication 1, dans lequel Y représente O, R⁶ représente l'hydrogène et R⁴ représente cycloalkyle en C₃-C₈.

3. Composé selon la revendication 2, ledit composé étant :
• le (2H-tétrazol-5-yl)-amide de l'acide 4-cycloheptyloxy-2-morpholin-4-yl-pyrimidine-5-carboxylique ;
• le (2H-tétrazol-5-yl)-amide de l'acide 4-cyclopentyloxy-2-morpholin-4-yl-pyrimidine-5-carboxylique ;
• le (2H-tétrazol-5-yl)-amide de l'acide 4-cyclohexyloxy-2-morpholin-4-yl-pyrimidine-5-carboxylique ;
• le (2H-tétrazol-5-yl)-amide de l'acide 4-cyclooctyloxy-2-morpholin-4-yl-pyrimidine-5-carboxylique ;
• le (2H-tétrazol-5-yl)-amide de l'acide 4-(2-cyclohexyléthoxy)-2-morpholin-4-yl-pyrimidine-5-carboxylique ;
• le (2H-tétrazol-5-yl)-amide de l'acide 4-cyclohexylméthoxy-2-morpholin-4-yl-pyrimidine-5-carboxylique ;
• le (2H-tétrazol-5-yl)-amide de l'acide 4-(3,3-diméthylcyclohexyloxy)-2-morpholin-4-yl-pyrimidine-5-carboxylique ; et
• le (2H-tétrazol-5-yl)-amide de l'acide 4-(3,5-diméthylcyclohexyloxy)-2-morpholin-4-yl-pyrimidine-5-carboxylique.

4. Composé selon la revendication 1, ledit composé étant sélectionné dans le groupe consistant en :
• le (2H-tétrazol-5-yl)-amide de l'acide 4-(adamantan-2-yloxy)-2-morpholin-4-yl-pyrimidine-5-carboxylique ; et
• le (2H-tétrazol-5-yl)-amide de l'acide 4-(bicyclo[2.2.1]-hept-2-yloxy)-2-morpholin-4-yl-pyrimidine-5-carboxylique.

5. Composé selon la revendication 1, dans lequel Y représente O, R⁶ représente l'hydrogène et R⁴ représente cycloalkyle en C₃-C₈ ou (alkylène en C₁-C₃)-cycloalkyle en C₃-C₈.

6. Composé selon la revendication 1, dans lequel Y représente O, R⁶ représente l'hydrogène et R⁴ représente hétérocycloalkyle ayant 5 ou 6 éléments.

7. Composé selon la revendication 1, dans lequel R⁴ représente tétrahydropyranyle.

8. Composé selon la revendication 1, dans lequel Y représente O, R⁶ représente l'hydrogène et R⁴ représente un groupe phényle.

9. Composé selon la revendication 8, ledit composé étant sélectionné dans le groupe consistant en :
• le (2H-tétrazol-5-yl)-amide de l'acide 4-(4-isopropylphénoxy)-2-morpholin-4-yl-pyrimidine-5-carboxylique ; et
• le (2H-tétrazol-5-yl)-amide de l'acide 4-(4-cyclohexylphénoxy)-2-morpholin-4-yl-pyrimidine-5-carboxylique.

10. Composé selon la revendication 1, dans lequel Y représente NH et R⁶ représente l'hydrogène.

11. Utilisation d'un composé selon la revendication 1 dans la fabrication d'une composition pharmaceutique comprenant une quantité thérapeutiquement efficace d'un composé selon la revendication 1 et un support pharmaceutiquement acceptable pour le traitement d'une maladie sélectionnée dans le groupe consistant en :
l'arthrite rhumatoïde, la spondylite ankylosante, l'ostéoarthrite, l'arthrite psoriatique, le psoriasis, les maladies inflammatoires et les maladies auto-immunes.

12. Utilisation selon la revendication 11, dans laquelle ledit composé est un composé selon l'une quelconque des revendications 1 à 10.

13. Composition pharmaceutique comprenant :
une quantité thérapeutiquement efficace d'un composé selon la revendication 1 et un support pharmaceutiquement acceptable.

14. Composition pharmaceutique comprenant :
une quantité thérapeutiquement efficace d'un composé selon l'une quelconque des revendications 1 à 10 et un support pharmaceutiquement acceptable.

15. Composition pharmaceutique comprenant une quantité thérapeutiquement efficace d'un composé sélectionné dans le groupe consistant en :
• le (2H-tétrazol-5-yl)-amide de l'acide 4-cycloheptyloxy-2-morpholin-4-yl-pyrimidine-5-carboxylique ;
• le (2H-tétrazol-5-yl)-amide de l'acide 4-cyclopentyloxy-2-morpholin-4-yl-pyrimidine-5-carboxylique ;
• le (2H-tétrazol-5-yl)-amide de l'acide 4-cyclohexyloxy-2-morpholin-4-yl-pyrimidine-5-carboxylique ;
• le (2H-tétrazol-5-yl)-amide de l'acide 4-cyclooctyloxy-2-morpholin-4-yl-pyrimidine-5-carboxylique ;
• le (2H-tétrazol-5-yl)-amide de l'acide 4-(2-cyclohexyléthoxy)-2-morpholin-4-yl-pyrimidine-5-carboxylique ;
• le (2H-tétrazol-5-yl)-amide de l'acide 4-cyclohexylméthoxy-2-morpholin-4-yl-pyrimidine-5-carboxylique ;
• le (2H-tétrazol-5-yl)-amide de l'acide 4-(3,3-diméthylcyclohexyloxy)-2-morpholin-4-yl-pyrimidine-5-carboxylique ;
• le (2H-tétrazol-5-yl)-amide de l'acide 4-(3,5-diméthylcyclohexyloxy)-2-morpholin-4-yl-pyrimidine-5-carboxylique ;
• le (2H-tétrazol-5-yl)-amide de l'acide 4-(adamantan-2-yloxy)-2-morpholin-4-yl-pyrimidine-5-carboxylique ;
• le (2H-tétrazol-5-yl)-amide de l'acide 4-(bicyclo[2.2.1]-hept-2-yloxy)-2-morpholin-4-yl-pyrimidine-5-carboxylique ;
• le (2H-tétrazol-5-yl)-amide de l'acide 4-(4-isopropylphénoxy)-2-morpholin-4-yl-pyrimidine-5-carboxylique-; et
• le (2H-tétrazol-5-yl)-amide de l'acide 4-(4-cyclohexylphénoxy)-2-morpholin-4-yl-pyrimidine-5-carboxylique.
